# EUROPÄISCHE PATENTSCHRIFT

(11) **EP 0 764 467 B1**
(45) Veröffentlichungstag und Bekanntmachung des Hinweises auf die Patenterteilung: **05.02.2003**
(21) Anmeldenummer: 96114457.3
(22) Anmeldetag: 10.09.1996
(51) Int. Cl.: B01J 35/10, B01J 37/00

(54) **Monomodale und polymodale Katalysatorträger und Katalysatoren mit engen Porengrössenverteilungen und deren Herstellverfahren**
Narrow pore size distribution monomodal and polymodal catalysts and catalyst carriers, and their manufacture
Catalyseurs ou supports de catalyseur monomode et polymode à plage porométrique étroite et leur procédé de fabrication

(30) Priorität: 12.09.1995 DE 19533484
(43) Veröffentlichungstag der Anmeldung: 26.03.1997
(73) Patentinhaber: BASF AKTIENGESELLSCHAFT, 67056 Ludwigshafen (DE)
(72) Erfinder: Trübenbach, Peter, Dr., 67065 Ludwigshafen (DE); Hagemeyer, Alfred, Dr., 67063 Ludwigshafen (DE); Lauth, Günter, Prof. Dr., 23909 Ratzenburg (DE); Dingerdissen, Uwe, Dr., 64342 Seeheim-Jugenheim (DE); Bröcker, Franz Josef, Dr., 67061 Ludwigshafen (DE); Flick, Klemens, Dr., 76863 Herxheim (DE)
(74) Vertreter: Isenbruck, Günter, Dr.

(56) Entgegenhaltungen:
- EP-A- 0 349 223
- EP-A- 0 444 475
- EP-A- 0 603 990
- EP-A- 0 761 307
- DD-A- 292 149
- DE-A- 2 459 475
- DE-A- 4 128 629
- US-A- 4 032 433
- US-A- 4 314 913
- US-A- 5 191 144
- US-A- 5 322 821
- Hagen Jens, Technische Katalyse: eine Einführung; Weinheim, VCH, 1996, p.225
- Römpp Chemie Lexikon, 9. Auflage, 1992, Georg Thieme Verlag Stuttgart, p. 3587.

## Beschreibung

Die vorliegende Erfindung betrifft Katalysatorträger und Katalysatoren mit einer engen Porengrößenverteilung, Verfahren zu deren Herstellung und deren Verwendungen.

Aus der US-A-5,191,144 sind Zeolithe mit einer sehr einheitlichen im Mesoporenbereich (2 bis 50 mm) variierbaren Porengröße zwischen 2 und 10 mm durch Hydrothermalsynthese bekannt. Aufgrund der geringen Porengröße ist die Größe der umsetzbaren Moleküle beschränkt. Da bei Zeolithen ein SiO₂-Binder verwendet wird, sind diese Zeolithe nur bis 700°C stabil und über 400°C nicht chemikalienbeständig, und ihre mechanische Beständigkeit ist schlecht. Die Zeolithe haben darüberhinaus sehr saure Oberflächen, wodurch ihre Anwendung auf sauer katalysierte Reaktionen beschränkt ist.

Aus Chem. Ind., 10 (1993) 48 bis 49 ist ein Verfahren zur Herstellung von Katalysatorträgern im Mesoporenbereich (2 bis 50nm) aus pyrogenen Oxiden (SiO₂, Al₂O₃, TiO₂, ZrO₂) durch Umsetzung der Halogenide in der Knallgasflamme bekannt, deren Porengrößen zwischen 10 und 70nm liegen, wobei keine Poren im Bereich kleiner 8nm resultieren. Die Porenverteilungen sind jedoch breit gestreut.

Aus der Chem. Ing. Tech., 56 (1984) 455 bis 463 ist Melamin als Makroporenbildner bekannt. Die Pyrolyse führt jedoch zu Rißbildungen.

Aus der US-A-3,755,204 sind poröse Keramikkörper durch Formen einer Mischung aus Keramikpulver, einem Polyolefin (Polyethylencopolymer) und eines Weichmachers (Mineralöl, Diethylenglykol, Polyvinylalkohol, Polyvinylpyrrolidon) bekannt, die durch Pyrolyse und Sintern hergestellt werden. Die bei der Pyrolyse auftretenden Risse im Keramikkörper können durch Extraktion des Weichmachers mit wässrigen Tensid-Lösungen vermieden werden (DE-A-24 59 475). Hierbei handelt es sich um keramische Wabenkörper, bei denen die offenen Poren als Kanalstrukturen durch die thermoplastische Formgebung eingebracht werden.

Aus der DE-A-24 59 475 ist ein Verfahren zur Herstellung von geformten porösen Metallstrukturen bekannt, bei dem ein Gemisch aus Metallpulver, Polyolefin und einem Weichmacher compoundiert und der Weichmacher vor der Pyrolyse aus dem Gemisch mit einem Lösungsmittel herausgelöst wird.

Aus der DE-A-41 20 687 sind poröse Sinterteile aus Metall oder Keramik, mit einem Polyolefin, einem Weichmacher und einem Zusatzstoff, der nicht mit dem Bindersystem mischbar ist, bekannt. Der Zusatzstoff ist ein vinylaromatisches Copolymer auf Basis von Polystyrol, das mit einer Teilchengröße von 20 bis 250 µm als Platzhalter zwischen den Metall- oder Keramikteilchen eingebracht wird. Durch eine Variation der Zusatzstoff-Teilchengröße lassen sich unterschiedliche aber nicht spezifizierte Porengrößen herstellen. Die Porengrößenverteilungen ist bimodal und breit gestreut, da die Poren einerseits durch die Teilchengrößenverteilung des Zusatzstoffes und andererseits durch die Zwischenräume der Metall- oder Keramikteilchen gebildet werden.

Aus der EP-A-446 708 ist die Herstellung von dichten metallischen Formteilen und aus der EP-A-444 475 die Herstellung von dichten keramischen Formkörpern über die Verformung von thermoplastischen Massen bekannt.

Aus der EP-A-413 231 ist ein Verfahren zur Herstellung von dichten anorganischen Sinterformteilen bekannt, wobei Grünkörper aus sinterbaren Pulvern und Polyoxymethylen oder einem Copolymerisat mit überwiegendem Anteil an Oxymethyleinheiten als Binder durch Extrusion, Spritzgießen oder Strangpressen geformt werden. Das Bindemittel wird aus diesen Grünteilen durch Behandlung mit einer gasförmigen Säure oder Bortrifluorid schnell, rißfrei und verzugsfrei entfernt. Die entbinderten porösen Teile haben eine geringe mechanische Stabilität und werden zu vollständiger Dichte gesintert. Über diesen Weg werden kompliziert geformte, dichte, keramische oder metallische Strukturwerkstoffe oder Funktionswerkstoffe hergestellt, die sich nicht als Katalysatorträger oder Katalysatoren eignen.

Sinterbare organische Polymere, wie Teflon (EP-A-513 500), Polyamid oder nichtplastifizierbare Polymere (EP-A-517 025) können mit diesem Herstellverfahren auch zu dichten Bauteilen verarbeitet werden.

Aus der DE-A-41 20 687 ist bekannt, daß es sehr schwierig ist, mit den bekannten Verfahren aus sehr feinen Metall- oder Keramikpulvern (Teilchengröße ca. 1 µm) reproduzierbar mechanisch stabile Teile mit gleichbleibender Porenverteilung herzustellen.

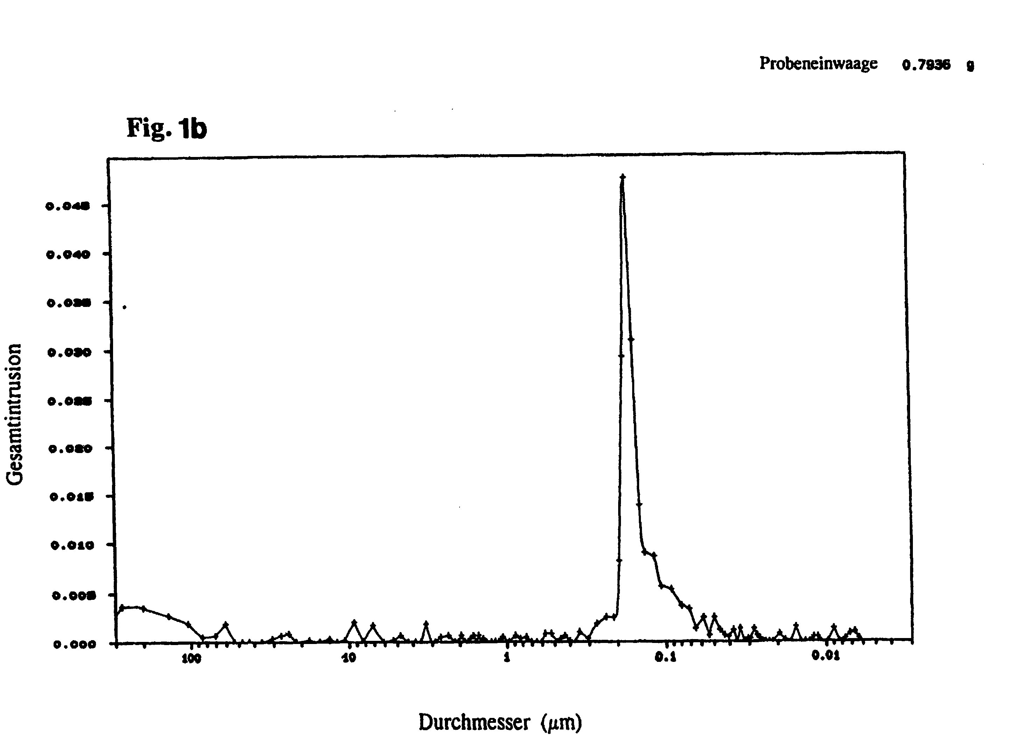

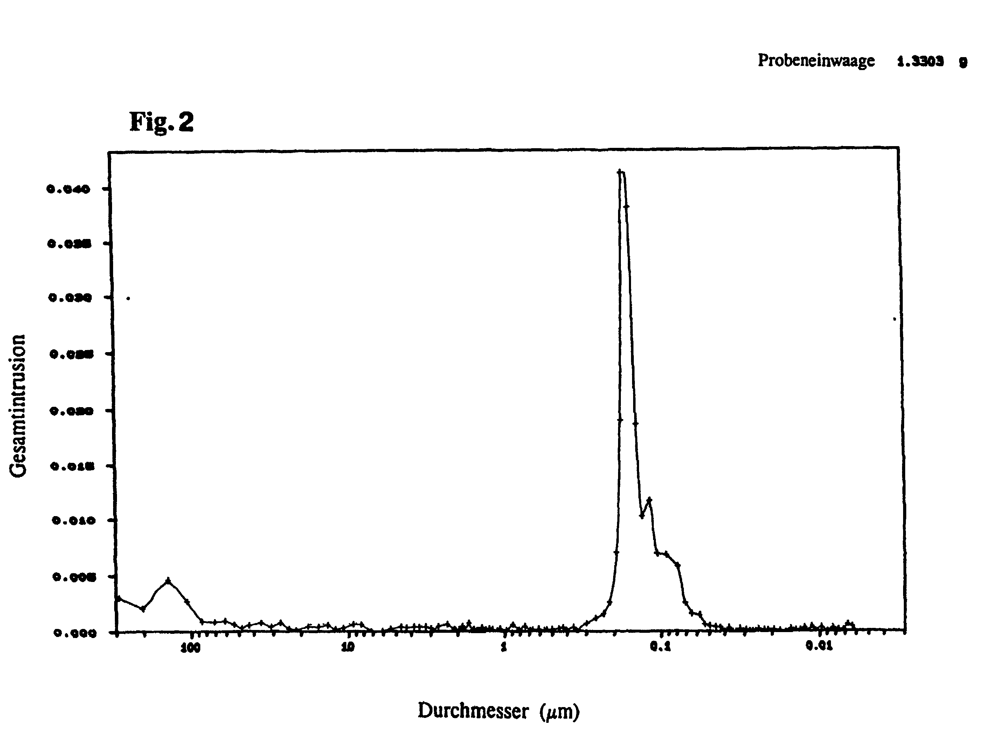

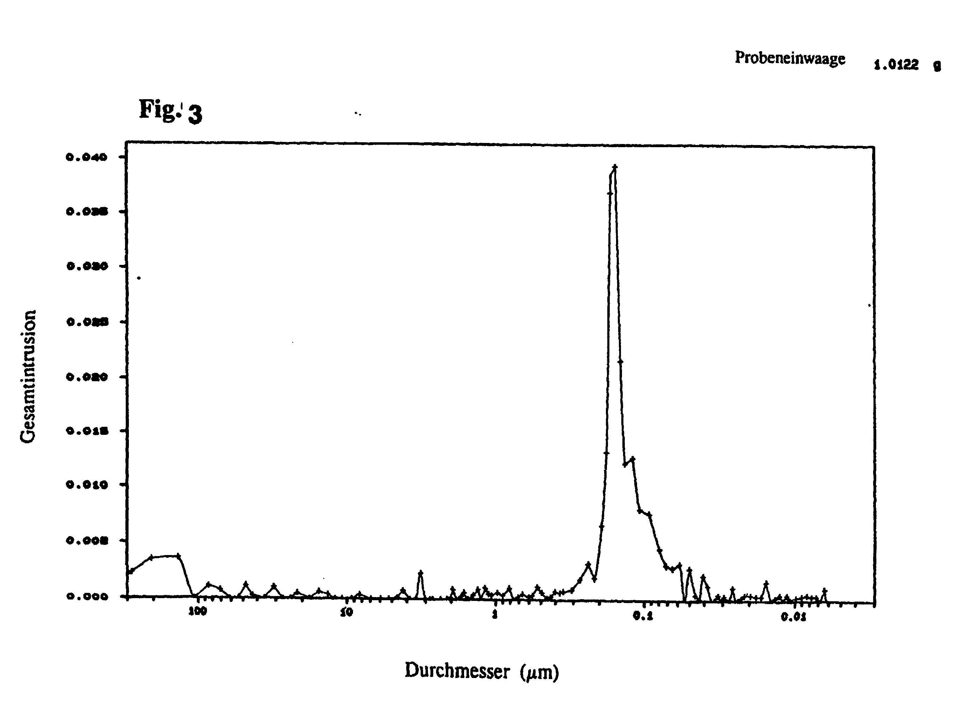

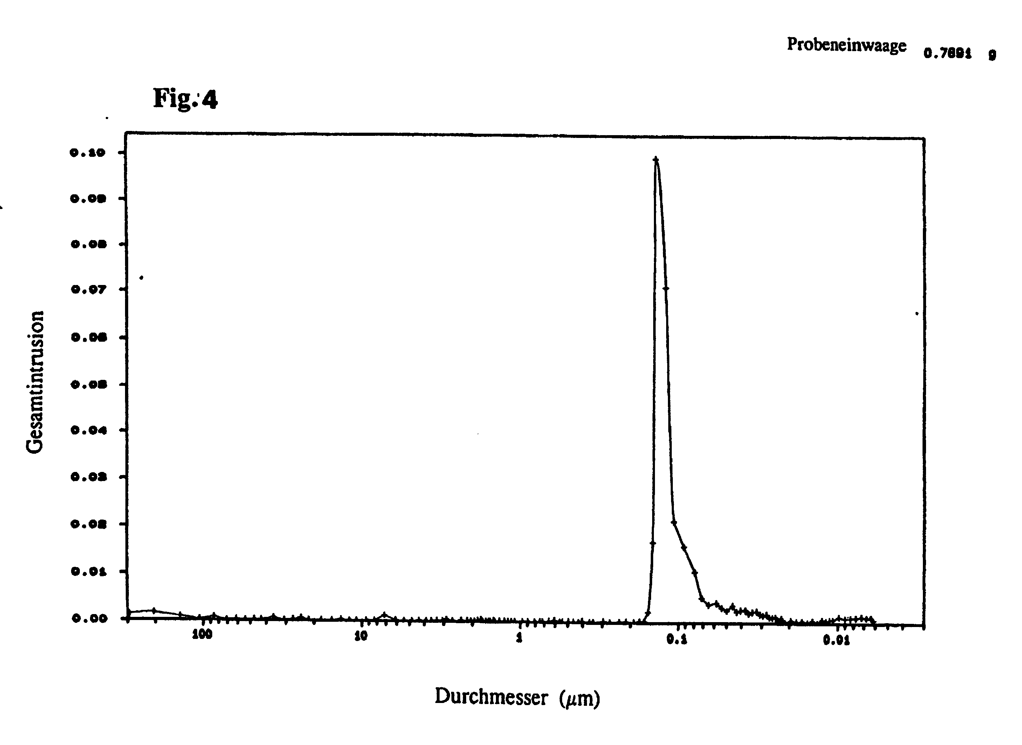

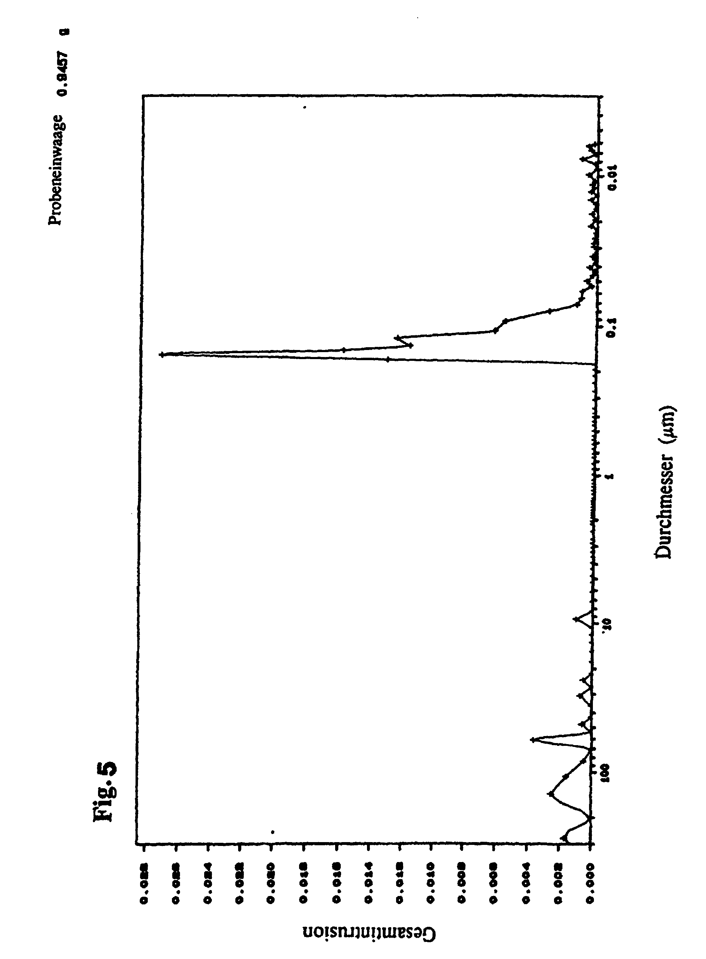

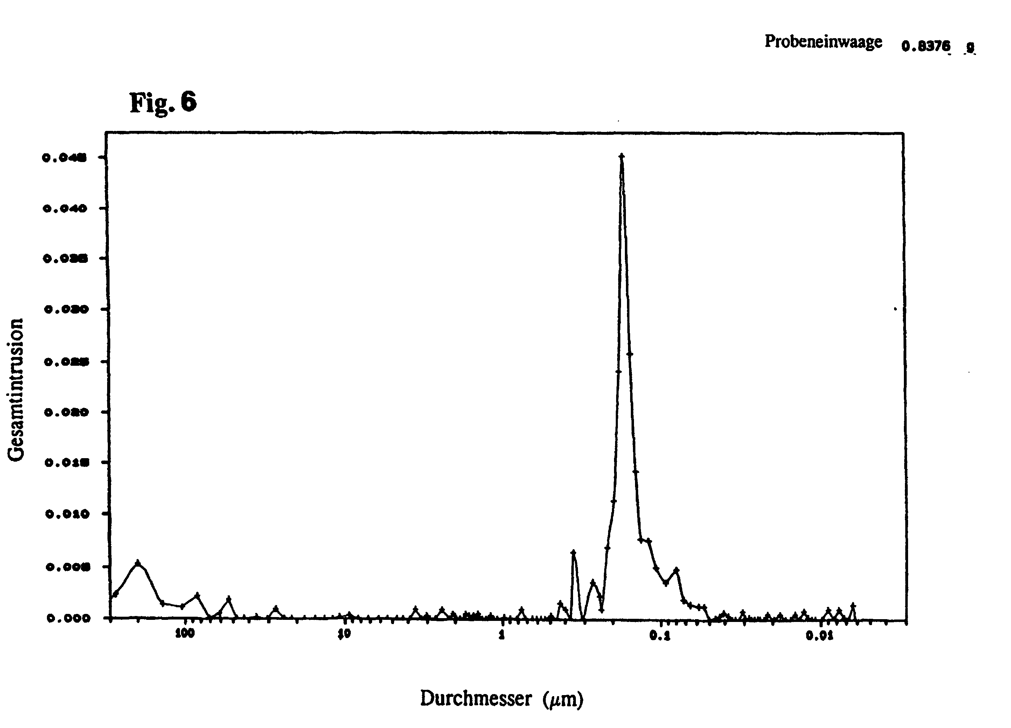

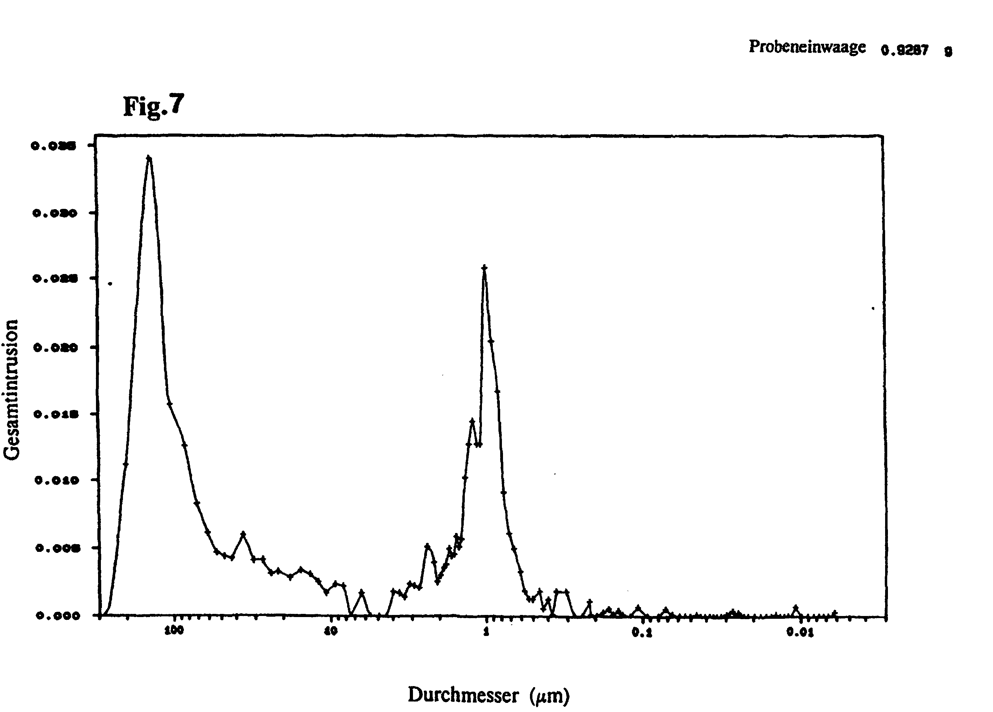

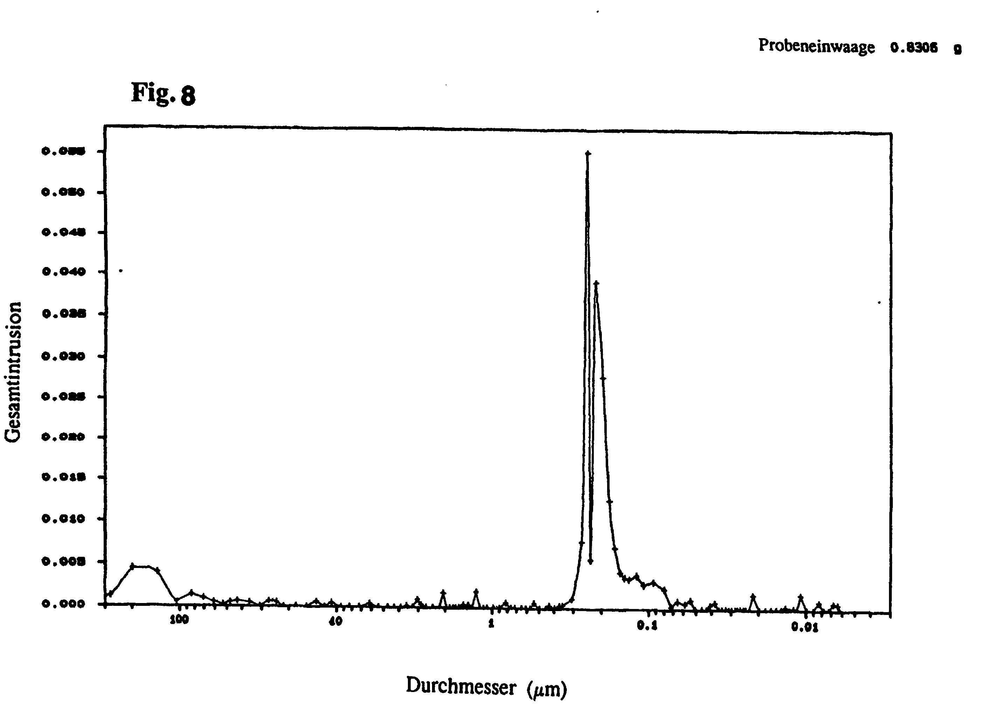

DE-A-41 28 629 betrifft silberhaltige Aluminiumoxidträger-Katalysatoren für die selektive Zersetzung von reinem oder in Gasgemischen enthaltenem Distickstoffmonoxid. Bei den Trägermaterialien handelt es sich um käufliches oder nach üblichen Methoden hergestelltes Aluminiumoxid mit einer BET-Oberfläche von 26 bis 350 m²/g. In den Figuren 1 und 2 sind die Porengrößenverteilungen eingesetzter handelsüblicher Aluminiumoxid-Träger dargestellt. Dabei handelt es sich um Trägermaterialien mit im wesentlichen bimodaler Porengrößenverteilung, wobei die Porengrößenverteilung verhältnismäßig breit ist.

Der vorliegenden Erfindung lag daher die Aufgabe zugrunde, den zuvor genannten Nachteilen abzuhelfen.

Demgemäß wurden neue und verbesserte monomodale oder polymodale Katalysatorträger oder Katalysatoren gefunden, welche dadurch gekennzeichnet sind, daß die Katalysatorträger oder Katalysatoren eine spezifische Oberfläche nach BET von 0,01 bis 250 m²/g, ein Porenvolumen von 0,05 bis 5 ml/g, eine Schneidhärte von 1-8 kg bei bei 800°C vorgesinterten Proben und eine monomodale oder eine polymodale Porengrößenverteilung mit einem mittleren Porendurchmesser von 50 bis 300.000nm
gemessen mit der Hg-Druckporosimetrie-Methode aufweisen und
a) 10 bis 95% des Porenvolumens bei dem 0,2- bis 100-fachen und/oder
b) 10 bis 80% des Porenvolumens bei dem 0,8- bis 100-fachen und/oder
c) 50 bis 95% des Porenvolumens bei dem 0,2- bis 1-fachen und/oder
d) 50 bis 80% des Porenvolumens bei dem 0,8- bis 1-fachen des mittleren Porendurchmessers liegt und
e) die Halbwertsbreite der Porengrößenverteilung weniger als das 0,6-fache des modalen Porendurchmessers beträgt, sowie
ein Verfahren zu deren Herstellung durch Verformen eines Gemisches aus
A) 15 bis 70 Vol.-% I) eines anorganischen Pulvers ausgewählt aus der Gruppe der Oxide, Nitride, Carbide, Silikate, Alumosilikate der Elemente Beryllium, Magnesium, Calcium, Strontium, Barium, Bor, Aluminium, Gallium, Indium, Thallium, Silicium, Germanium, Zinn, Blei, Antimon, Selen, Tellur, Polonium, Astat, Eisen, Cobalt, Nickel, Ruthenium, Rhodium, Palladium, Osmium, Iridium, Platin, Kupfer, Silber, Gold, Zink, Cadmium, Quecksilber, Scandium, Yttrium, Lanthan, Actinium, Titan, Zirkon, Hafnium, Vanadin, Niob, Tantal, Chrom, Molybdän, Wolfram, Mangan, Technetium, Rhenium, Cer, Praseodym oder deren Gemische und/oder
   II) eines metallischen Pulvers ausgewählt aus Metallen und Legierungen der Elemente Bor, Aluminium, Gallium, Indium, Thallium, Silicium, Germanium, Zinn, Blei, Antimon, Selen, Tellur, Polonium, Neodym, Samarium, Dysprosium, Astat, Eisen, Cobalt, Raney-Cobalt, Nickel, Raney-Nickel, Ruthenium, Rhodium, Palladium, Osmium, Iridium, Platin, Kupfer, Silber, Gold, Zink, Cadmium, Quecksilber, Scandium, Yttrium, Lanthan, Actinium, Titan, Zirkon, Hafnium, Vanadin, Niob, Tantal, Chrom, Molybdän, Wolfram, Mangan, Technetium, Rhenium, Cer, Praseodym, WC, TiC, TaC, VC oder deren Gemische, WC-Cobalt, TiCCobalt, TaC-Cobalt, VC-Cobalt oder deren Gemische sowie Kohlenstoff und/oder
   III) einer Aktivkomponerte ausgewählt aus der Gruppe der anorganischen Säuren, der Metalle ausgewählt aus Lithium, Natrium, Kalium, Rubidium, Caesium, Francium, Beryllium, Magnesium, Calcium, Strontium, Barium, Bor, Aluminium, Gallium, Indium, Thallium, Silicium, Germanium, Zinn, Blei, Arsen, Antimon, Wismut, Selen, Tellur, Polonium, Astat, Eisen, Cobalt, Raney-Cobalt, Nikkel, Raney-Nickel, Ruthenium, Rhodium, Palladium, Osmium, Iridium, Platin, Kupfer, Silber, Gold, Zink, Cadmium, Quecksilber, Scandium, Yttrium, Lanthan, Actinium, Titan, Zirkon, Hafnium, Vanadin, Niob, Tantal, Chrom, Molybdän, Wolfram, Mangan, Technetium, Rhenium, Cer, Praseodym, deren Gemische, oder deren Boraten, Carbonaten, Silikaten, Nitraten, Phosphaten, Arsenaten, Antimonaten, Bismutaten, Sulfaten, Selenaten, Telluraten, Vanadaten, Molybdaten, Niobaten, Chromaten, Oxiden, Hydroxiden, Halogeniden, Sulfiden, Seleniden, Telluriden, Nitriden, Phosphiden, Arseniden, Acetate, Acetylacetonate, Palladate, Platinate, Cyanide, Rhodanide, Manganate, Rhenate, Osmate, Carbiden, Siliciden, Boriden, deren Ammoniumverbindungen oder deren Gemische und/oder
   IV) eines organischen Pulvers ausgewählt aus der Gruppe Teflon oder Polyimid
B) 30 bis 85 Vol.-% eines Polyethylen- oder Polypropylenpolymers oder eines Copolymeren aus Ethylen, Propylen, Buten-1 oder Isobuten oder eines Polystyrolcopolymers oder eines Polymethylmethacrylatcopolymers oder eines Polyethylenoxidcopolymers oder eines Ethylenvinylacetatcopolymers oder einer Mischung aus
   B₁) 50 bis 100 Gew.-% eines Polyoxymethylenhomo- oder copolymerisats und
   B₂) 0 bis 50 Gew.-% eines in B₁) homogen gelösten oder mit einer mittleren Teilchengröße von weniger als 1µm in B₁) dispergierten Polymerisats und
C) 0 bis 15 Vol.-% eines Dispergierhilfsmittels,
   Entfernen des Bindemittels und anschließendes Vorsintern und gegebenenfalls Aufbringen von Aktivkomponenten III) auf der Komponente A) oder auf der vorgesinterten Masse durch gegebenenfalls mehrfaches Tränken, Imprägnieren, Sprühimprägnieren, Auffällen, Hicoaten, Washcoaten oder Sprühtrocknen, indem die Komponente A) ein nanokristallines Pulver mit einer mittleren Korngröße von 5 nm bis 500.000 nm ist, wobei die Abweichungen der Korngröße bei 80% der Körner 0 bis 30% der mittleren Korngröße betragen und das Bindemittel durch Pyrolyse bei Temperaturen von 300 bis 600°C entfernt wird und bei Temperaturen von 600 bis 1400°C vorgesintert wird und die Katalysatorträger oder Katalysatoren nach der pyrolytischen Entfernung des Binders eine spezifische Oberfläche nach BET von 0,01 bis 250 m²/g und eine Porengrößenverteilung von 50 bis 300.000 nm gemessen mit der Hg-Druckporosimetrie-Methode aufweisen, sowie deren Verwendung zur Chlorherstellung aus Chlorwasserstoff in einem instationären Deacon-Prozeß, zur Umsetzung von Ethylbenzol zu Styrol in einer instationären Oxy-Dehydrierung, zur Aziridinherstellung aus Ethanolamin, zur Umsetzung von Trimethylcyclohexenon zu Trimethylphenol, bei Reduktionen, Hydrierungen, Oxidationen, Dehydrierungen, sauer oder basisch katalysierten Reaktionen oder Reaktionen in der Wirbelschicht, zur Entfernung von Verbrennungsrückständen aus Dieselabgasen und zur Entfernung von NOₓ aus Abgasen, in Bioreaktoren gemeinsam mit Bakterien und als Biokatalysatorträger mit immobilisierten Enzymen oder Mikroben.

Die erfindungsgemäßen Katalysatorträger oder Katalysatoren sind bevorzugt nicht zeolithisch und haben eine spezifische Oberfläche nach BET von 0,01 bis 250 m²/g, bevorzugt 0,1 bis 200 m²/g, besonders bevorzugt 0,5 bis 120 m²/g und eine monomodale oder eine polymodale, also eine bimodale, trimodale, tetramodale oder höher modale, bevorzugt eine bimodale, trimodale oder tetramodale, besonders bevorzugt eine bimodale oder trimodale Porengrößenverteilung mit einem mittleren Porendurchmesser von 50 bis 300.000nm, bevorzugt 100 bis 50.000nm, besonders bevorzugt 150 bis 25.000nm gemessen mit der Hg-Druckporosimetrie-Methode aufweisen und
a) 10 bis 95%, bevorzugt 30 bis 95%, besonders bevorzugt 50 bis 95% des Porenvolumens bei dem 0,2- bis 100-fachen und/oder
b) 10 bis 80%, bevorzugt 30 bis 80%, besonders bevorzugt 50 bis 80% des Porenvolumens bei dem 0,8- bis 100-fachen und/oder
c) 50 bis 95% , bevorzugt 70 bis 95%, besonders bevorzugt 80 bis 95% des Porenvolumens bei dem 0,2- bis 1-fachen und/oder
d) 50 bis 80%, bevorzugt 60 bis 80%, besonders bevorzugt 65 bis 80% des Porenvolumens bei dem 0,8- bis 1-fachen des mittleren Porendurchmessers liegt und
e) die Halbwertsbreite der Porengrößenverteilung weniger als das 0,6-fache, also das 0,001- bis 0,59-fache, bevorzugt das 0,005- bis 0,4-fache, besonders bevorzugt das 0,1-bis 0,35-fache des modalen Porendurchmessers beträgt.

Unter den erfindungsgemäßen Katalysatorträgern oder Katalysatoren sind diejenigen bevorzugt, bei denen die Bedingungen a) und b) oder a) und c) oder a) und d) oder b) und c) oder b) und d) oder c) und d) gleichzeitig erfüllt sind, besonders bevorzugt sind diejenigen, bei denen die Bedingungen a), b) und c) oder a), b) und d) oder a), c) und d) oder a), c) und d) oder b), c) und d) gleichzeitig erfüllt sind, insbesondere diejenigen Katalysatorträger oder Katalysatoren, bei denen alle vier Bedingungen a), b), c) und d) gleichzeitig erfüllt sind.

Die erfindungsgemäßen Katalysatorträger oder Katalysatoren können wie folgt hergestellt werden:

In einer Mischvorrichtung, bevorzugt mit Heizvorrichtung, beispielsweise einem Kneter, einem Extruder oder einem Scherwalzenextruder kann dem Polymer der Komponente B) in geschmolzenem Zustand bei Temperaturen von 80 bis 250°C, bevorzugt 100 bis 220°C, besonders bevorzugt 120 bis 200°C die Komponente A), die anorganischen, metallischen, organischen Pulver und/oder die Aktivkomponenten und dann das Dispergierhilfsmittel der Komponente C) oder zuerst Komponente C) und dann Komponente A) oder die Komponenten A) und C) gemeinsam zugegeben werden. Die innig (intensiv) vermischten Massen können z.B. durch Granulieren, Pressen, Walzen,

Extrusion, Verstrangen oder Spritzgießen, insbesondere durch Spritzgießen, bei Temperaturen von 120 bis 250°C, bevorzugt 140 bis 220°C, besonders bevorzugt 150 bis 200°C und Drücken von 500 bis 2000 bar, bevorzugt 600 bis 1800 bar, besonders bevorzugt 700 bis 1600 bar verformt werden. Dabei können in einem Formgebungsschritt bei Spritzgießformtemperaturen von 40 bis 160°C, bevorzugt 60 bis 150°C, besonders bevorzugt 80 bis 140°C, beliebig geformte Katalysatorträger oder Katalysatoren als Schüttung von Einzelteilen oder als Monolithe wie beispielsweise Raschig-Ringe, Sattelkörper, Sternringe, gelochte und/oder gerippte geometrische Körper wie Ringe, Kugeln, Quader, Würfel, Kegel, Pyramiden, Prismen, Oktaeder, Zylinder, Pyramidenstümpfe und Kegelstümpfe, in der Regel ohne Nachbearbeitung hergestellt werden.

Wagenräderprofile, Honigwabenprofile und Fensterrahmenprofile können bei Temperaturen von 120 bis 280°C, besonders bevorzugt bei 180 bis 200°C zu Monolithen extrudiert werden.

Die nach dem Formgebungsvorgang erhaltenen Grünkörper können durch Pyrolyse bei 300 bis 600°C, bevorzugt 350 bis 600°C, besonders bevorzugt 400 bis 600°C behandelt und durch anschließendes Vorsintern, bei Temperaturen von 600 bis 1400°C, bevorzugt 600 bis 1100°C, besonders bevorzugt bei 600 bis 800°C unter oxidierenden Bedingungen (Luft), Inertgas (N₂, Ar, He) oder reduzierenden Bedingungen (N₂/H₂, Ar/H₂) zu den Katalysatorträgern oder Katalysatoren mit ihrer endgültigen Festigkeit und Porenverteilung umgewandelt werden. Durch den Vorsinterprozeß erhöht sich in der Regel die Stabilität und die Härte der porösen Formkörper beträchtlich. Die Schneidehärte der bei 800°C vorgesinterten Proben beträgt 1 bis 8 kg (800°C), bevorzugt 1,5 bis 7 kg (800°C), besonders bevorzugt 2 bis 6 kg (800°C). Bei mit 1100°C vorgesinterten Proben wurden Schneidhärten von bis zu 20 kg erreicht. Die Wasseraufnahme liegt im Bereich von 0,05 bis 5 ml/g, bevorzugt 0,1 bis 3 ml/g, besonders bevorzugt 0,1 bis 1 ml/g, so daß mehr Aktivkomponente auf einen erfindungsgemäßen Katalysatorträger aufgebracht werden kann, ohne in der Regel wesentliche Einbußen in der Härte zu erzielen. Durch die ausgezeichnete Aktivkomponentenaufnahme können die Katalysatoren nach Gebrauch durch Nachtränken der Aktivkomponenten gut recycliert werden. Neben streng monomodalen Porengrößenverteilungen können so auch polymodale (bimodale, trimodale, tetramodale und höher modale) Porengrößenverteilungen hergestellt werden. Mit diesem Verfahren können Katalysatorträger und Katalysatoren mit einer hohen Festigkeit und einer hohen thermischen bzw. chemischen Stabilität hergestellt werden. Als Geometrien der Formkörper sind alle Formen denkbar, die über Granulierung, Walzen, Pressen, Strangpressen, Extrusion oder Spritzguß herstellbar sind. Die Formkörper können als Schüttgut oder in Form von Monolithen bei katalytischen Reaktionen eingesetzt werden.

Die spezifischen Oberflächen nach BET der erfindungsgemäßen Katalysatorträger und Katalysatoren liegen bei 0,01 bis 250 m²/g, bevorzugt 0,1 und 150 m²/g, besonders bevorzugt 1 und 100 m²/g, insbesondere 2 bis 8 m²/g (800°C).

Die mittlere Porengröße wird in der Regel von der Korngröße der Komponente A), der anorganischen, metallischen, organischen Pulver und/oder der Aktivkomponenten in der Regel nur durch die Zwischenräume der eingesetzten Pulverteilchen bestimmt. Die mittlere Porengröße und die Porengrößenverteilung sind deshalb von der mittleren Korngröße und der Teilchengrößenverteilung des eingesetzten Pulvers abhängig. Mit kommerziell erhältlichen Metall- oder Keramikpulvern können auf diese Weise mechanisch stabile, rißfreie, monomodal oder polymodal poröse Materialien, wie die erfindungsgemäßen Katalysatorträger oder Katalysatoren, hergestellt werden. Die enge Porengrößenverteilung kann somit je nach Bedarf im Meso- und Makroporenbereich eingestellt werden und führt in der Regel zu einer hoch monodispersen Porenverteilung.

Sofern polymodale Katalysatorträger oder Katalysatoren erhalten werden sollen, setzt man Pulver mit polymodaler Korngrößenverteilung oder mit innerer Porosität ein.

Die mittlere Korngröße der erfindungsgemäß eingesetzten Pulver der Komponente A) ist ein nanokristallines Pulver von 5 nm bis 500.000nm, bevorzugt 300 nm bis 100.000 nm, besonders bevorzugt 500 nm bis 50.000 nm, wobei die Abweichungen der Korngröße bei 80%, bevorzugt 90%, besonders bevorzugt 95% der Körner 0 bis 30%, bevorzugt 0 bis 20%, besonders bevorzugt 0 bis 10% von der mittleren Korngröße beträgt.

Als Komponente A) eignen sich:
I) eines anorganischen Pulvers ausgewählt aus der Gruppe der Oxide, Nitride, Carbide, Silikate, Alumosilikate der Elemente Beryllium, Magnesium,Calcium, Strontium, Barium, Bor, Aluminium, Gallium, Indium, Thallium, Silicium, Germanium, Zinn, Blei, Antimon, Selen, Tellur, Polonium, Astat, Eisen, Cobalt, Nickel, Ruthenium, Rhodium, Palladium, Osmium, Iridium, Platin, Kupfer, Silber, Gold, Zink, Cadmium, Quecksilber, Scandium, Yttrium, Lanthan, Actinium, Titan, Zirkon, Hafnium, Vanadin, Niob, Tantal, Chrom, Molybdän, Wolfram, Mangan, Technetium, Rhenium, Cer, Praseodym oder deren Gemische, bevorzugt Oxide, Nitride, Carbide, Silikate der Elemente Magnesium, Calcium, Strontium, Barium, Bor, Aluminium, Silicium, Zinn, Blei, Antimon, Eisen, Cobalt, Nickel, Ruthenium, Rhodium, Palladium, Platin, Kupfer, Silber, Gold, Zink, Yttrium, Lanthan, Titan, Zirkon, Vanadin, Niob, Tantal, Chrom, Molybdän, Wolfram, Mangan, Cer oder deren Gemische, besonders bevorzugt Oxide, Nitride, Carbide, der Elemente Magnesium, Calcium, Strontium, Barium, Bor, Aluminium, Silicium, Zinn, Antimon, Eisen, Cobalt, Nickel, Kupfer, Yttrium, Titan, Zirkon, Vanadin, Niob, Tantal, Chrom, Molybdän, Wolfram, Mangan oder deren Gemische,
II) eines metallischen Pulvers ausgewählt aus Metallen und Legierungen der Elemente Bor, Aluminium, Gallium, Indium, Thallium, Silicium, Germanium, Zinn, Blei, Antimon, Selen, Tellur, Polonium, Neodym, Samarium, Dysprosium, Astat, Eisen, Cobalt, Raney-Cobalt, Nickel, Raney-Nickel, Ruthenium, Rhodium, Palladium, Osmium, Iridium, Platin, Kupfer, Silber, Gold, Zink, Cadmium, Quecksilber, Scandium, Yttrium, Lanthan, Actinium, Titan, Zirkon, Hafnium, Vanadin, Niob, Tantal, Chrom, Molybdän, Wolfram, Mangan, Technetium, Rhenium, Cer, Praseodym, WC, TiC, TaC, VC oder deren Gemische, WC-Cobalt, TiC-Cobalt, TaC-Cobalt, VC-Cobalt oder deren Gemische sowie Kohlenstoff, bevorzugt Metallen und Legierungen der Elemente Bor, Aluminium, Silicium, Zinn, Blei, Antimon, Selen, Neodym, Samarium, Dysprosium, Eisen, Cobalt, Raney-Cobalt, Nickel, Raney-Nickel, Palladium, Platin, Kupfer, Silber, Gold, Zink, Yttrium, Lanthan, Titan, Zirkon, Vanadin, Niob, Tantal, Chrom, Molybdän, Wolfram, Mangan, Cer, WC, TiC, TaC, VC oder deren Gemische, WC-Cobalt, TiC-Cobalt, TaC-Cobalt, VC-Cobalt oder deren Gemische sowie Kohlenstoff, besonders bevorzugt Metallen und Legierungen der Elemente Bor, Aluminium, Silicium, Zinn, Neodym, Samarium, Dysprosium, Eisen, Cobalt, Raney-Cobalt, Nickel, Raney-Nickel, Kupfer, Zink, Yttrium, Titan, Zirkon, Vanadin, Niob, Tantal, Chrom, Molybdän, Wolfram, Mangan, WC, TiC, TaC, VC oder deren Gemische, WC-Cobalt, TiC-Cobalt, TaC-Cobalt, VC-Cobalt oder deren Gemische,
III) einer Aktivkomponente ausgewählt aus der Gruppe der anorganischen Säuren, insbesondere H₂SO₄, H₃PO₄, HNO₃ und Heteropolysäuren, der Metalle ausgewählt aus Lithium, Natrium, Kalium, Rubidium, Caesium, Francium, Beryllium, Magnesium, Calcium, Strontium, Barium, Bor, Aluminium, Gallium, Indium, Thallium, Silicium, Germanium, Zinn, Blei, Arsen, Antimon, Bismut, Selen, Tellur, Polonium, Astat, Eisen, Cobalt, Raney-Cobalt, Nickel, Raney-Nickel, Ruthenium, Rhodium, Palladium, Osmium, Iridium, Platin, Kupfer, Silber, Gold, Zink, Cadmium, Quecksilber, Scandium, Yttrium, Lanthan, Actinium, Titan, Zirkon, Hafnium, Vanadin, Niob, Tantal, Chrom, Molybdän, Wolfram, Mangan, Technetium, Rhenium, Cer, Praseodym, deren Gemische, oder deren Borate, Carbonate, Silikate, Nitrate, Phosphate, Arsenate, Antimonate, Bismutate, Sulfate, Selenate, Tellurate, Vanadate, Molybdate, Niobate, Chromate, Oxide, Hydroxide, Halogenide, Sulfide, Selenide, Telluride, Nitride, Phosphide, Arsenide, Acetate, Acetylacetonate, Palladate, Platinate, Cyanide, Rhodanide, Manganate, Rhenate, Osmate, Carbide, Silicide, Boride, deren Ammoniumverbindungen oder deren Gemische, bevorzugt Metalle ausgewählt aus Lithium, Natrium, Kalium, Caesium, Magnesium, Calcium, Strontium, Barium, Bor, Aluminium, Silicium, Zinn, Blei, Arsen, Antimon, Bismut, Selen, Tellur, Polonium, Eisen, Cobalt, Raney-Cobalt, Nickel, Raney-Nickel, Ruthenium, Rhodium, Palladium, Osmium, Iridium, Platin, Kupfer, Silber, Gold, Zink, Yttrium, Lanthan, Titan, Zirkon, Vanadin, Niob, Tantal, Chrom, Molybdän, Wolfram, Mangan, Rhenium, Cer, deren Gemische, oder deren Borate, Carbonate, Silikate, Nitrate, Phosphate, Arsenate, Antimonate, Bismutate, Sulfate, Selenate, Vanadate, Molybdate, Niobate, Chromate, Oxide, Hydroxide, Halogenide, Sulfide, Selenide, Telluride, Nitride, Phosphide, Arsenide, Acetate, Acetylacetonate, Palladate, Platinate, Manganate, Carbiden, Siliciden, Boriden, deren Ammoniumverbindungen oder deren Gemische, besonders bevorzugt Metalle ausgewählt aus Lithium, Natrium, Kalium, Caesium, Magnesium, Calcium, Strontium, Barium, Aluminium, Silicium, Zinn, Blei, Arsen, Antimon, Bismut, Eisen, Cobalt, Nickel, Ruthenium, Rhodium, Palladium, Platin, Kupfer, Silber, Zink, Titan, Zirkon, Vanadin, Niob, Tantal, Chrom, Molybdän, Wolfram, Mangan, Cer, deren Gemische, oder deren Carbonate, Silikate, Nitrate, Phosphate, Arsenate, Antimonate, Bismutate, Sulfate, Vanadate, Molybdate, Niobate, Chromate, Oxide, Hydroxide, Halogenide, Sulfide, Nitride, Carbide, deren Ammoniumverbindungen oder deren Gemische.

Vorzugsweise werden als Metall Aluminium, Eisen, Cobalt, Nickel, Palladium, Platin, Kupfer, Silber, Molybdän, Zink, Titan, Zirkon, Wolfram, Niob, Chrom oder Kohlenstoff oder als anorganisches Pulver Al₂O₃, MgO, SiO₂, TiO₂, Y₂O₃, ZrO₂, ZnO, Fe₃O₄, F₂O₃, CoO, Co₂O₃, Cr₂O₃, NiO, B₂O₃, Ce₂O₃, CeO₂, Pr₂O₃, B₄C, SiC, WC, TiC, TaC, Si₃N₄, AlN, BN, TiN, ZrN oder deren Gemische, weiter bevorzugt als Metall Eisen, Cobalt, Nickel, Chrom, Molybdän, Titan oder als anorganisches Pulver SiC, Si₃N₄, BN, B₄C, WC, TiC, TiN, ZrN und AlN, oder deren Gemische und insbesondere als anorganisches Pulver SiC, Si₃N₄ oder deren Gemische eingesetzt.

Sollen Vollkatalysatoren hergestellt werden, so beinhaltet die Komponente A) nur Aktivkomponenten III) und ggf. IV), eines organischen Pulvers ausgewählt aus der Gruppe Teflon oder Polyimid.

Als Komponente B) eignen sich:

Polyethylen- oder Polypropylenpolymere oder Copolymere aus Ethylen, Propylen, Buten-1 oder Isobuten oder Polystyrolcopolymere oder Polymethylmethacrylatcopolymere oder Polyethylenoxidcopolymere oder Ethylenvinylacetatcopolymere oder Mischungen aus
B₁) 50 bis 100 Gew.-%, bevorzugt 70 bis 90 Gew.-%, besonders bevorzugt 80 bis 88 Gew.-% eines Polyoxymethylenhomo- oder copolymerisats wie sie aus EP-A-444 475 bekannt sind und
B₂) 0 bis 50 Gew.-%, bevorzugt 10 bis 30 Gew.-%, besonders bevorzugt 12 bis 25 Gew.-% eines in B₁) homogen gelösten oder mit einer mittleren Teilchengröße von weniger als 1µm in B₁) dispergierten Polymerisats, bevorzugt Poly-1,3-dioxolan, Poly-1,3-dioxan, Poly-1,3-dioxepan, besonders bevorzugt Poly-1,3-dioxepan.

Der organische Binder kann auch aus Mischungen ein oder mehrerer thermoplastischen Harzen, wie Polyacetal, Polyethylen, Polypropylen, Polystyrol, Polymethylmethacrylat und ein oder mehreren Weichmachern, wie Polyethylenglykol, Polypropylenglykol, Polybutandiolformal, Phtalsäureestern, Ethylen-Vinylacetat-Copolymeren und Montanesterwachsen bestehen.

Als Polyacetalbinder eignet sich beispielsweise Polyoxymethylen, das vorteilhaft eine Molmasse von 10.000 bis 500.000 aufweist. Neben Homopolymerisaten von Formaldehyd oder Trioxan kommen auch Copolymerisate aus Trioxan mit z.B. cyclischen Ethern wie Ethylenoxid und 1,3-Dioxolan oder Formalen wie 1,3-Dioxepan, 1,3-Dioxan, oder deren Mischungen oder homopolymeres Poly-1,3-dioxolan, Poly-1,3-dioxan, oder Poly-1,3-dioxepan in Betracht, wobei die Mengen der Copolymeren im allgemeinen bei 10 bis 30 Gew.-% der Polymeren liegen.

Außerdem können sie Hilfsmittel, wie thermoplastische Binder, wie Polyethylen, Polymethylmethacrylat oder Polyethylenoxid und Dispergatoren bzw. Schmiermittel, wie Polyethylenglykol, Stearinsäure, Fettalkohole, Polyvinyl pyrrolidin oder Polyvinylalkohol enthalten. Die Menge an Hilfsmittel liegt in der Regel zwischen 0,1 und 12 Gew.-% der Gesamtmasse.

Als Komponente C) eignen sich Dispergierhilfsmittels wie sie aus EPA-0 444 475 bekannt sind, beispielsweise organische Carbonsäuren, Amine, Amide oder Maleinimide, Stearinsäure, Polyvinylpyrrolidon, Polyvinylalkohol, Polyethylenglykol, Polypropylenglykol, Polyethylenoxid und Montanwachse, bevorzugt organische Carbonsäuren, Amine, Amide oder Maleinimide, Polyethylenglykol und Polyethylenoxid, besonders bevorzugt organische Carbonsäuren, Amine, Maleinimide, Polyethylenglykol und Polyethylenoxid.

Die zur Herstellung (Zusammenmischung) der erfindungsgemäßen Katalysatorträger oder Katalysatoren eingesetzten Gemische enthalten oder bestehen in der Regel aus 15 bis 70 Gew.-%, bevorzugt 30 bis 70 Gew.-%, besonders bevorzugt 50 bis 65 Gew.-% Komponente A), aus 30 bis 85 Gew.-%, bevorzugt 30 bis 70 Gew.-%, besonders bevorzugt 35 bis 50 Gew.-% Komponente B) und aus 0 bis 15 Gew.-%, bevorzugt 1 bis 12 Gew.-%, besonders bevorzugt 2 bis 8 Gew.-% Komponente C).

Als Trägermaterialien eignen sich keramische, metallische oder Pulver aus A I, II und IV.

Die Aktivkomponenten können im Falle des Vollkatalysators direkt als Pulver eingesetzt oder im Falle von Trägerkatalysatoren auf das anorganische Pulver I), das metallische Pulver II), das organische Pulver IV) oder deren Gemische, oder nachträglich auf das Trägermaterial aufgebracht, oder zusammen mit dem Trägermaterial compoundiert werden.

Weiterhin können den Massen anorganische oder organische Fasern oder Whisker aus z.B. Al₂O₃, SiO₂, SiC, Si₃N₄, C oder deren Gemische zugesetzt werden.

Bei dem erfindungsgemäßen Herstellungsverfahren werden in der Regel mit Hilfe eines Dispergators C) die Komponenten A) desagglomeriert und damit die einheitlich großen Ausgangspulverpartikel mit einem vergleichsweisen hohen Füllgrad in einen organischen Binder [Komponente B)] eingearbeitet. Der organische Binder füllt die in der Regel nahezu einheitlich großen und regelmäßig angeordneten Zwischenräume der Pulverpartikel. Die bei dem Ausgangspulver der Komponente A) durch Agglomeratbildung vorhandenen Makroporen im Bereich um 100µm werden durch die Desagglomerierung in der Regel aufgehoben. Nach der Entfernung des organischen Binders und des organischen Dispergators bleiben bei Einsatz von Pulver mit einer engen monomodalen Korngrößenverteilung streng einheitlich große Poren zwischen den Pulverteilchen zurück. In der Regel beträgt der mittlere Porendurchmesser 25% des mittleren Korndurchmessers des eingesetzten Pulvers (s. Tabelle [P5-P-Tab dm/KG]). Beim Einsatz von Pulvern mit polymodaler Korngrößenverteilung oder beim Einsatz von porösen Pulvern können auch polymodale (bimodale, trimodale, tetramodale oder höhermodale) Porenverteilungen hergestellt werden, wobei die Porengröße durch die Zwischenräume zwischen den Pulverteilchen und durch die innere Porosität der Pulverteilchen bedingt ist.

Die erfindungsgemäßen Katalysatoren können heterogene Trägerkatalysatoren oder Vollkatalysatoren sein. Vollkatalysatoren bestehen aus katalytisch aktivem Material. Trägerkatalysatoren können durch Beschichtung von inerten porösen keramischen oder metallischen Katalysatorträgern mit katalytisch aktiven Komponenten oder Precursoren von katalytisch aktiven Komponenten durch Tränken, Imprägnieren, Sprühimprägnieren, Sprühtrocknen, Auffällen, Hicoaten, Washcoaten hergestellt werden.

Die Erfindung betrifft auch monomodale oder polymodale Katalysatorträger oder Katalysatoren, herstellbar durch Verformen eines Gemisches aus
A) 15 bis 70 Vol.-%
   I) eines anorganischen Pulvers ausgewählt aus der Gruppe der Oxide, Nitride, Carbide, Silikate, Alumosilikate der Elemente Beryllium, Magnesium, Calcium, Strontium, Barium, Bor, Aluminium, Gallium, Indium, Thallium, Silicium, Germanium, Zinn, Blei, Antimon, Selen, Tellur, Polonium, Astat, Eisen, Cobalt, Nickel, Ruthenium, Rhodium, Palladium, Osmium, Iridium, Platin, Kupfer, Silber, Gold, Zink, Cadmium, Quecksilber, Scandium, Yttrium, Lanthan, Actinium, Titan, Zirkon, Hafnium, Vanadin, Niob, Tantal, Chrom, Molybdän, Wolfram, Mangan, Technetium, Rhenium, Cer, Praseodym oder deren Gemische und/oder
   II) eines metallischen Pulvers ausgewählt aus Metallen und Legierungen der Elemente Bor, Aluminium, Gallium, Indium, Thallium, Silicium, Germanium, Zinn, Blei, Antimon, Selen, Tellur, Polonium, Neodym, Samarium, Dysprosium, Astat, Eisen, Cobalt, Raney-Cobalt, Nickel, Raney-Nickel, Ruthenium, Rhodium, Palladium, Osmium, Iridium, Platin, Kupfer, Silber, Gold, Zink, Cadmium, Quecksilber, Scandium, Yttrium, Lanthan, Actinium, Titan, Zirkon, Hafnium, Vanadin, Niob, Tantal, Chrom, Molybdän, Wolfram, Mangan, Technetium, Rhenium, Cer, Praseodym, WC, TiC, TaC, VC oder deren Gemische, WC-Cobalt, TiC-Cobalt, TaC-Cobalt, VC-Cobalt oder deren Gemische sowie Kohlenstoff und/oder
   III) einer Aktivkomponente ausgewählt aus der Gruppe der anorganischen Säuren, der Metalle ausgewählt aus Lithium, Natrium, Kalium, Rubidium, Caesium, Francium, Beryllium, Magnesium, Calcium, Strontium, Barium, Bor, Aluminium, Gallium, Indium, Thallium, Silicium, Germanium, Zinn, Blei, Arsen, Antimon, Wismut, Selen, Tellur, Polonium, Astat, Eisen, Cobalt, Raney-Cobalt, Nikkel, Raney-Nickel, Ruthenium, Rhodium, Palladium, Osmium, Iridium, Platin, Kupfer, Silber, Gold, Zink, Cadmium, Quecksilber, Scandium, Yttrium, Lanthan, Actinium, Titan, Zirkon, Hafnium, Vanadin, Niob, Tantal, Chrom, Molybdän, Wolfram, Mangan, Technetium, Rhenium, Cer, Praseodym, deren Gemische, oder deren Boraten, Carbonaten, Silikaten, Nitraten, Phosphaten, Arsenaten, Antimonaten, Bismutaten, Sulfaten, Selenaten, Telluraten, Vanadaten, Molybdaten, Niobaten, Chromaten, Oxiden, Hydroxiden, Halogeniden, Sulfiden, Seleniden, Telluriden, Nitriden, Phosphiden, Arseniden, Acetate, Acetylacetonate, Palladate, Platinate, Cyanide, Rhodanide, Manganate, Rhenate, Osmate, Carbiden, Siliciden, Boriden, deren Ammoniumverbingungen oder deren Gemische und/oder
   IV) eines organischen Pulvers ausgewählt aus der Gruppe Teflon oder Polyimid
B) 30 bis 85 Vol.-% eines Polyethylen- oder Polypropylenpolymers oder eines Copolymeren aus Ethylen, Propylen, Buten-1 oder Isobuten oder eines Polystyrolcopolymers oder eines Polymethylmethacrylatcopolymers oder eines Polyethylenoxidcopolymers oder eines Ethylenvinylacetatcopolymers oder einer Mischung aus
   B₁) 50 bis 100 Gew.-% eines Polyoxymethylenhomo- oder copolymerisats und
   B₂) 0 bis 50 Gew.-% eines in B1) homogen gelösten oder mit einer mittleren Teilchengröße von weniger als 1µm in B1) dispergierten Polymerisats und
C) 0 bis 15 Vol.-% eines Dispergierhilfsmittels, Entfernen des Bindemittels durch Pyrolyse bei Temperaturen von 300 bis 600°C und anschließendes Vorsintern bei Temperaturen von 600 bis 1400°C und gegebenenfalls Aufbringen von Aktivkomponenten III auf der Komponente A) oder auf der vorgesinterten Masse durch gegebenenfalls mehrfaches Tränken, Imprägnieren, Sprühimprägnieren, Auffällen, Hicoaten, Washcoaten oder Sprühtrocknen, dadurch gekennzeichnet, daß die Katalysatorträger oder Katalysatoren nach der pyrolytischen Entfernung des Binders eine spezifische Oberfläche nach BET von 0,01 bis 250m²/g und eine Porengrößenverteilung von 50 bis 300.000nm gemessen mit der Hg-Druckporosimetrie-Methode aufweisen.

Die erfindungsgemäßen Katalysatorträger oder Katalysatoren eignen sich in der Regel für den Einsatz bei:
- Reduktionen (Hydrierungen), beispielsweise: Hydrierung von Alkinen wie z.B. die selektive Hydrierung von Acetylen in C₂-, C₃-, C₄-Gemischen, die selektive Hydrierung von Vinylacetylen in C₄-Schnitten und die Hydrierung von Butindiol zu Butendiol oder Butandiol, die Hydrierungen von Alkenen wie z.B. die Hydrierung ungesättigter Verbindungen bei der Oxo-Synthese, die aminierende Hydrierung, die Aromatenhydrierung, die Diolefinhydrierung wie die Hydrierung von Diolefinen im Pyrolysebenzin, die Fetthydrierung, die hydrierende Entschwefelung wie die Hydrierung von anorganischen Schwefelverbindungen, z.B. COS, CS₂, SO₂ und Sₓ zu Schwefelwasserstoff, die hydrierende Raffination von Aromaten oder Paraffinen, die Hydrierung organischer Chlorverbindungen, die Hydrierung von Aldehyden, Carbonsäuren, Carbonsäureestern, Ketonen, Nitrilen, Nitroverbindungen, Oximen und Oxo-Produkten wie die Reduktion von Nitrobenzol zu Anilin, die Hydrierung von Carbonylgruppen und Aromaten z.B. zur Weißölerzeugung, die Hydrierung von Trimethylchinon zu Trimethylhydrochinon, die Hydrierung von Adipodinitril zu Hexamethylendiamin, Acrylnitril, NH₃ und die Hydrierung von Adipinsäure zu Hexandiol, die Hydrierung von Cyclohexylhydroperoxid zu Cyclohexanol, die Hydrierung von Citral zu Citronellal, die Herstellung von Lilial aus Dehydrolilial, die Entfernung von NOₓ aus Abgasen durch Reduktion mit Ammoniak oder Alkanen und die Herstellung von Alkanen, Olefinen, Alkoholen, Aldehyden und/oder Carbonsäuren aus Synthesegas; die Hydrierung von Adipodinitril zu Aminocapronitril, die aminierende Hydrierung von Adipinsäure zu Aminocapronitril;
- Oxidationen (Dehydrierungen), beispielsweise: Oxidationen von Alkanen wie die Dehydrierung von Ethylbenzol zu Styrol oder Dimethylcyclohexylamin zu 2,6-Dimethylanilin, von Alkenen, von Alkoholen wie die Dehydrierung von Cyclohexanol zu Cyclohexanon und die Herstellung von Ethylhexansäure und Ethylhexanal aus Ethylhexenol, die Ammonoxidation wie die Herstellung von Cyanwasserstoff aus Methan oder von o-Xylol zu Phthalodinitril, von Aromaten, die Epoxidation, die oxidative Halogenierung, die oxidative Kupplung, Oxidation von schwefelwasserstoffhaltigen Gasen zu Schwefel nach dem Claus-Verfahren, die Herstellung von Vinylchlorid nach dem Oxychlorierungsprozeß (Stauffer-Verfahren), die Oxidation von Schwefelwasserstoff und/oder organischen Schwefelverbindungen zu Schwefeldioxid, die Herstellung von Schwefelsäure nach dem "Kontaktverfahren" aus SO₂-haltigen Gasen, die Herstellung von Phthalsäureanhydrid aus o-Xylol und Luft, die katalytische Verbrennung von Kohlenwasserstoffen, Lösungsmitteln bzw. CO-verunreinigtem Abgas, die Herstellung von Ethylendichlorid durch Oxychlorierung von Ethylen, die Oxidation von Propen zu Acrylsäure, die Herstellung von Methacrylsäure aus Methacrolein, die Herstellung von Methacrylsäure aus Isobuttersäure, die Dehydrierung von DMCHA zu Xylidin und die Dehydrierung von Trimethylcyclohexenon zu Trimethylphenol, die Oxidation von Ethylen zu Ethylenoxid, die Oxidation von Butadien zu Furan, die Oxidation von Propen zu Furan, die Oxidation von Acrolein zu Acrylsäure, die Oxidation von Methacrolein zu Metharoylsäure;
- sauer oder basisch katalysierte Reaktionen, beispielsweise: Alkoxylierungen z.B. von Ethylenoxid oder Propylenoxid, Dealkoxylierungen z.B. von N-Vinylformamid aus α-Methoxiethylformamid, Alkylierungen, Acylierungen, Hydratisierungen, Dehydratisierungen z.B. von Aziridin aus Ethanolamin oder von Blausäure aus Formamid, Aminierungen, Aldolreaktionen, Oligomerisierungen, Polymerisationen, Polymeranaloge Reaktionen, Cyclisierungen, Isomerisierungen, Veresterungen, Spaltung gasförmiger Kohlenwasserstoffe (Cracking) z.B. Erdgas mit Wasserdampf und gegebenenfalls CO₂, die Oxidation von Propen zu Acrolein, Eliminierungsreaktion wie N-Formylalninnitril zu N-Vinylformamid, Addition wie Methanol und Propin zu α-Methoxypropen.

Darüberhinaus eignen sich makroporöse Träger mit einer Porengröße zwischen 100 und 10.000 nm zur Entfernung von Verbrennungsrückständen (Ruß) aus Dieselabgasen und für Bioreaktoren in Zusammenhang mit der Verwendung von Bakterien (1 bis 2 µm).

Die nach dem erfindungsgemäßen Verfahren hergestellten Katalysatorträger oder Katalysatoren haben vergleichsweise hohe mechanische Festigkeiten und sind deshalb besonders für Wirbelschichtreaktionen geeignet.

Als Wirbelschichtreaktionen kommen z.B. in Frage für die Umlagerung von Cyclohexanonoxim zu ∈-Caprolactam, die Ammonoxidationen, z.B. Toluol zu Benzonitril oder Propen zu Acrylnitril, die Herstellung von Maleinsäureanhydrid aus Buten oder die Herstellung von Anilin aus Nitrobenzol.

Demgemäß betrifft die vorliegende Erfindung auch die Verwendung der erfindungsgemäßen monomodalen oder polymodalen Katalysatorträger oder Katalysatoren zur Chlorherstellung aus Chlorwasserstoff in einem instationären Deacon-Prozeß, zur Umsetzung von Ethylbenzol zu Styrol in einer instationären Oxy-Dehydrierung, zur Aziridinherstellung aus Ethanolamin, zur Umsetzung von Trimethylcyclohexenon zu Trimethylphenol, bei Reduktionen, Hydrierungen, Oxidationen, Dehydrierungen, sauer oder basisch katalysierten Reaktionen oder Reaktionen in der Wirbelschicht, zur Entfernung von Verbrennungsrückständen aus Dieselabgasen und zur Entfernung von NOₓ aus Abgasen, in Bioreaktoren gemeinsam mit Bakterien und als Biokatalysatorträger mit immobilisierten Enzymen oder Mikroben.

### Beispiele

### Beispiel 1

Das Keramikpulver 1 aus Tabelle I wurde mit einem Binder auf Polyacetalbasis, bestehend aus einem Polyoxymethylencopolymer (POM/PBDF) aus Trioxan und 2,5 Gew.-% Butandiolformal mit einem durchschnittlichen Molekulargewicht von 150.000, sowie mit 20 Gew.-% Polybutandiolformal mit einem Molekulargewicht von 50.000 und 5 Gew.-% (bezogen auf das eingesetzte Pulver) Polyethylenglykol mit einem Molekulargewicht von 800 als Hilfsmittel, bei 180°C verknetet, zu Strängen mit einem Durchmesser von 4 mm bei 180°C extrudiert, die bei 600°C 1 h unter N₂ pyrolysiert und danach bei verschiedenen Temperaturen im Muffelofen an Luft vorgesintert. Die Verfahrensparameter sind Tabelle II, die Porenradienverteilungen und weitere Eigenschaften der Stränge sind Tabellen IIIa und IIIb zu entnehmen.

### Beispiel 2

Das Keramikpulver 2 aus Tabelle I wurde unter Zusatz von Polyethylen mit einer Molmasse von 150.000 und einer Dichte von 0.95g/ml und Polyethylenoxid mit einer Molmasse von 400 in einem Doppelsigmakneter bei 220°C compoundiert. Über eine Austragsschnecke wurde der Feedstock granuliert. Das Granulat wurde bei einer Temperatur von 600°C 1h unter N₂ pyrolysiert und danach im Muffelofen bei 800°C 2h an Luft vorgesintert. Die Verfahrensparameter sind Tabelle II, die Porenradienverteilungen und weitere Eigenschaften der Stränge sind Tabellen IIIa und IIIb zu entnehmen.

### Beispiel 3

Das Keramikpulver 3 aus Tabelle I wurde unter Zusatz von Polystyrol mit einer Molmasse von 100.000 und einer Dichte von 1,04 g/ml und Polyethylenoxid mit einer Molmasse von 400 in einem Doppelsigmakneter bei 200°C compoundiert. Über eine Austragsschnecke wurde der Feedstock granuliert. Das Granulat wurde bei einer Temperatur von 500°C 0,5 h unter N₂ pyrolysiert und danach bei 800°C 0,5 h mit 50 l/h Luft im Drehrohrofen vorgesintert. Die Verfahrensparameter sind Tabelle II, die Porenradienverteilungen und weitere Eigenschaften der Stränge sind Tabellen IIIa und IIIb zu entnehmen.

### Beispiel 4

Das Keramikpulver 4 aus Tabelle I mit 90 Gew.-% Si₃N₄ (HC STARCK, LC12) und 5 Gew.-% Y₂O₃ (HC STARCK, grade fine) und 5 Gew.-% Al₂O₃ (ALCOA, CT3000SG) wurde unter Zusatz von Polymethylmethacrylat mit einer Molmasse von 150.000 und einer Dichte von 1,17 g/ml und Polyethylenoxid mit einer Molmasse von 400 in einem Doppelsigmakneter bei 200°C compoundiert. Über eine Austragsschnecke wurde der Feedstock granuliert. Das Granulat wurde bei einer Temperatur von 600°C 1 h unter N₂ pyrolysiert und danach bei 600°C 2 h an Luft im Muffelofen vorgesintert. Die Verfahrensparameter sind Tabelle II, die Porenradienverteilungen und weitere Eigenschaften der Stränge sind Tabellen IIIa und IIIb zu entnehmen.

### Beispiel 5

Das Keramikpulver 5 aus Tabelle I wurde mit einem Binder auf Polyacetalbasis, bestehend aus einem Polyoxymethylencopolymer (POM/PBDF) aus Trioxan und 2,5 Gew.-% Butandiolformal mit einem durchschnittlichen Molekulargewicht von 150.000, sowie mit 20 Gew.-% Polybutandiolformal mit einem Molekulargewicht von 50.000 und 5 Gew.-% (bez. auf das eingesetzte Pulver) Polyethylenglykol mit einem Molekulargewicht von 800 als Hilfsmittel, bei 180°C verknetet, zu Strängen mit einem Durchmesser von 4 mm bei 180°C extrudiert, die bei 600°C unter N₂ pyrolysiert und danach bei 800°C 2 h im Muffelofen vorgesintert wurden. Die Verfahrensparameter sind Tabelle II, die Porenradienverteilungen und weitere Eigenschaften der Stränge sind Tabellen IIIa und IIIb zu entnehmen.

### Beispiel 6

Das Keramikpulver 6 aus Tabelle I wurde mit einem Binder auf Polyacetalbasis, bestehend aus einem Polyoxymethylencopolymer (POM/PBDF) aus Trioxan und 2,5 Gew.-% Butandiolformal mit einem durchschnittlichen Molekulargewicht von 150.000, sowie mit 20 Gew.-% Polybutandiolformal mit einem Molekulargewicht von 50.000 und 5 Gew.-% (bez. auf das eingesetzte Pulver) Polyethylenglykol mit einem Molekulargewicht von 800 als Hilfsmittel, bei 180°C verknetet, zu Strängen mit einem Durchmesser von 4 mm bei 180°C extrudiert, die in einem Muffelofen zunächst bei 600°C 1h unter Stickstoff pyrolysiert und danach bei 1100°C 2 h an Luft vorgesintert wurden. Die Verfahrensparameter sind Tabelle II, die Porenradienverteilungen und weitere Eigenschaften der Stränge sind Tabellen IIIa und IIIb zu entnehmen.

### Beispiel 7

Das Keramikpulver 7 aus Tabelle I mit einer bimodalen Korngrößenverteilung mit Maxima bei 1µm wurde mit einem Binder auf Polyacetalbasis, bestehend aus einem Polyoxymethylencopolymer (POM/PBDF) aus Trioxan und 2,5 Gew.-% Butandiolformal mit einem durchschnittlichen Molekulargewicht von 150.000, sowie mit 20 Gew.-% Polybutandiolformal mit einem Molekulargewicht von 50.000 und 5 Gew.-% (bez. auf das eingesetzte Pulver) Polyethylenglykol mit einem Molekulargewicht von 800 als Hilfsmittel, bei 180°C verknetet, zu Strängen mit einem Durchmesser von 4 mm bei 180°C extrudiert, die in einem Muffelofen bei 600°C 1 h unter Stickstoff pyrolysiert und danach bei 800°C 2 h an Luft vorgesintert wurden. Die Verfah-rensparameter sind Tabelle II, die Porenradienverteilungen und weitere Eigenschaften der Stränge sind Tabellen IIIa und IIIb zu entnehmen.

### Beispiel 8

Das Keramikpulver 8 aus Tabelle I mit einer bimodalen Korngrößenverteilung mit Maxima bei 1 µm wurde mit einem Binder auf Polyacetalbasis, bestehend aus einem Polyoxymethylencopolymer (POM/PBDF) aus Trioxan und 2,5 Gew.-% Butandiolformal mit einem durchschnittlichen Molekulargewicht von 150.000, sowie mit 20 Gew.-% Polybutandiolformal mit einem Molekulargewicht von 50.000 und 5 Gew.-% (bez. auf das eingesetzte Pulver) Polyethylenglykol mit einem Molekulargewicht von 800 als Hilfsmittel, bei 180°C verknetet. Das erkaltete Produkt wurde in einer Messermühle granuliert. Das Granulat wurde bei 600°C 1 h unter Stickstoff pyrolysiert und danach bei 1100°C 2 h an Luft vorgesintert. Die Verfahrensparameter sind Tabelle II, die Porenradienverteilungen und weitere Eigenschaften der Stränge sind Tabellen IIIa und IIIb zu entnehmen.

**Tabelle I**

| **Bsp. Nr.** | **Pulver** | **Bezeichnung** | **Pulvergehalt [Vol.-%]** | **Pulvermenge [g]** | **Polyacetal [g]** | **PBDF [g]** | **PE [g]** | **PMMA [g]** | **PS [g]** | **PEG [g]** | **PEO [g]** |
|---|---|---|---|---|---|---|---|---|---|---|---|
| 1 | Al₂O₃ | ALCOA CT3000SG | 56 | 1000 | 162 | 41 | | | | 50 | |
| 2 | Al₂O₃ | ALCOA CT3000SG | 56 | 1000 | | | 169 | | | | 20 |
| 3 | Al₂O₃ | ALCOA CT3000SG | 56 | 1000 | | | | | 185 | | 20 |
| 4 | 90% Si₃N₄ | HC STARCK LC12 | 50 | 900 | | | | 334 | | | 20 |
| | 5% Y₂O₃ | HC STARCK grade fine | --- | 50 | --- | --- | --- | --- | --- | --- | --- |
| | 5% Al₂O₃ | ALCOA CT3000SG | --- | 50 | --- | --- | --- | --- | --- | --- | --- |
| 5 | ZrO₂ | TOSOH TZ-3YS | 50 | 1000 | 127 | 32 | | | | 50 | |
| 6 | Al₂O₃ | ALCOA CT3000SG | 56 | 1000 | 162 | 41 | | | | 50 | |
| 7 | Al₂O₃ | ALCOA Tabular | 50 | 1000 | 219 | 55 | | | | 50 | |
| 8 | Al₂O₃ | Norton FCP13NLC | 49 | 1000 | 295 | 74 | | | | 50 | |
| Polyacetal = Copolymer aus Trioxan und 2,5% Butandiolformal, Molmasse 150.000 PBDF = Polybutandiolformal, Molmasse 50.000 PE = Polyethylen, Molmasse 150.000, d=0,95g/ml PMMA = Polymethylmethacrylat, Molmasse 150.000, d=1,17g/ml, Lucryl G88 UV1 [BASF] PS = Polystyrol, Molmasse 100.000, d=1,04g/ml, 168N [BASF] PEG = Polyethylenglykol, Molmasse 800 PEO = Polyethylenoxid, Molmasse 400 | | | | | | | | | | | |

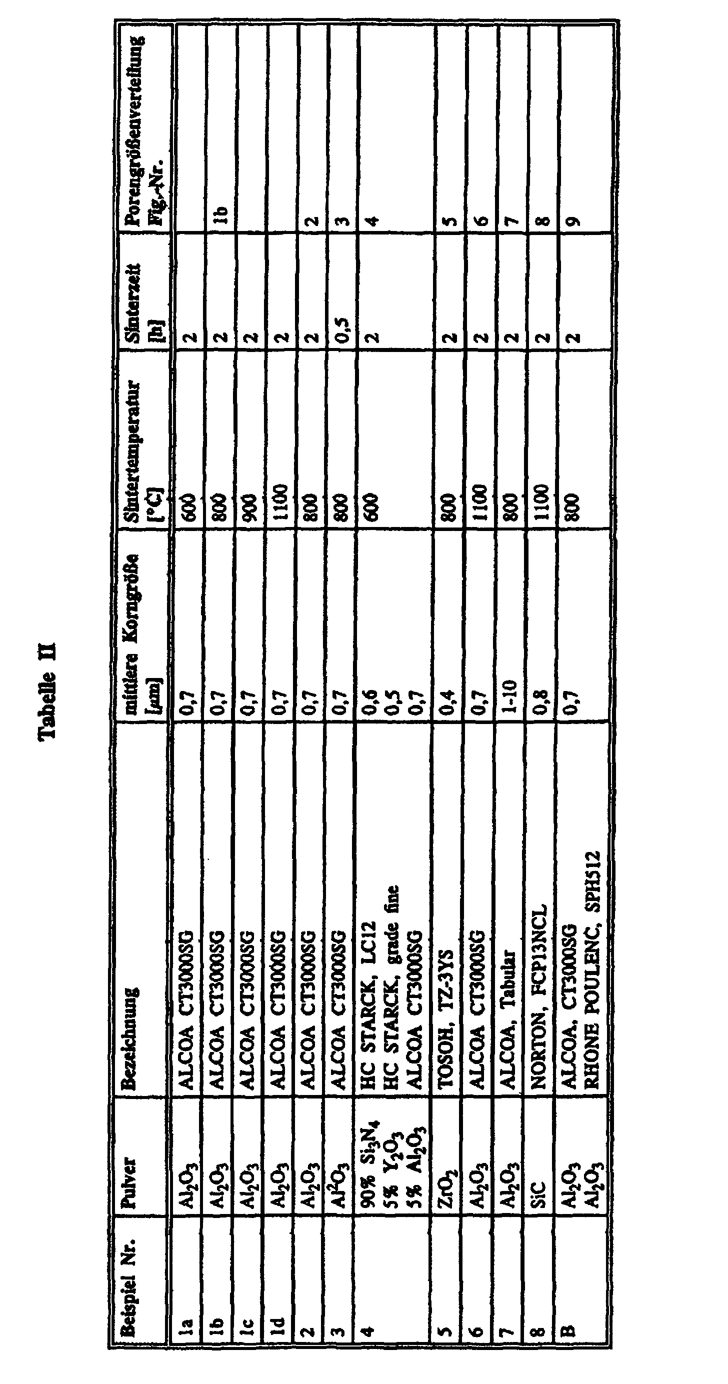

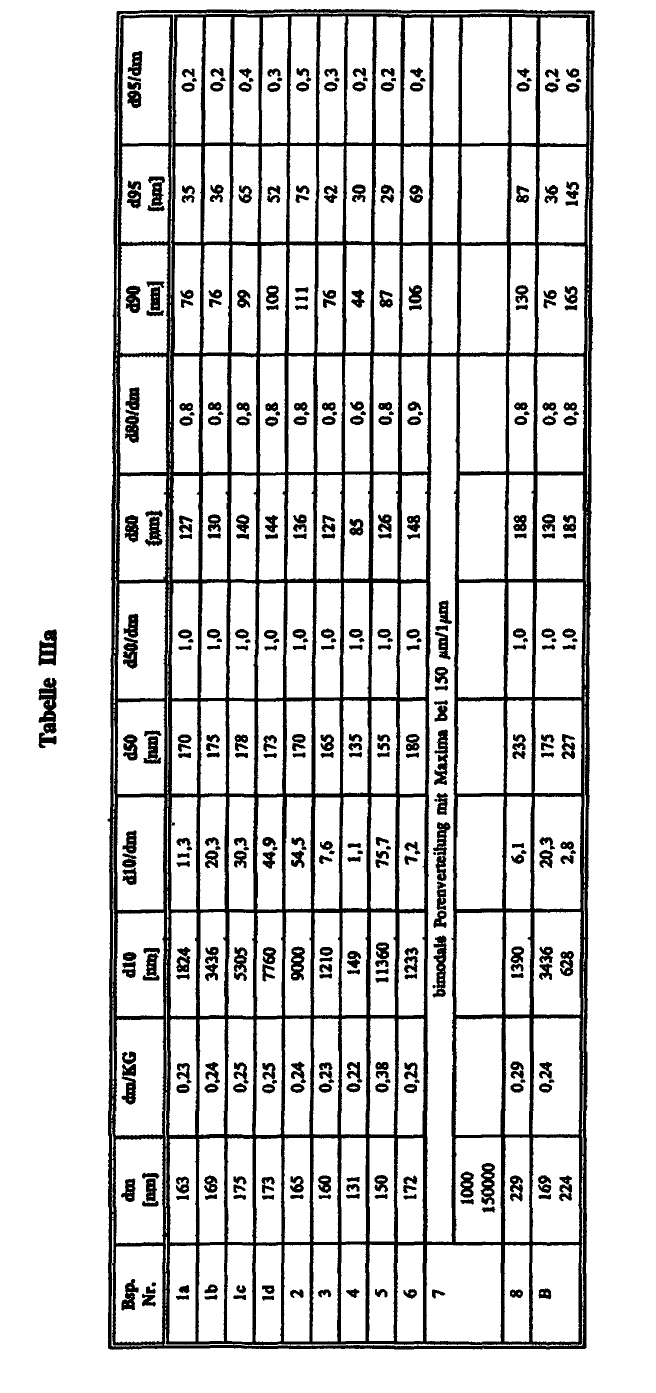

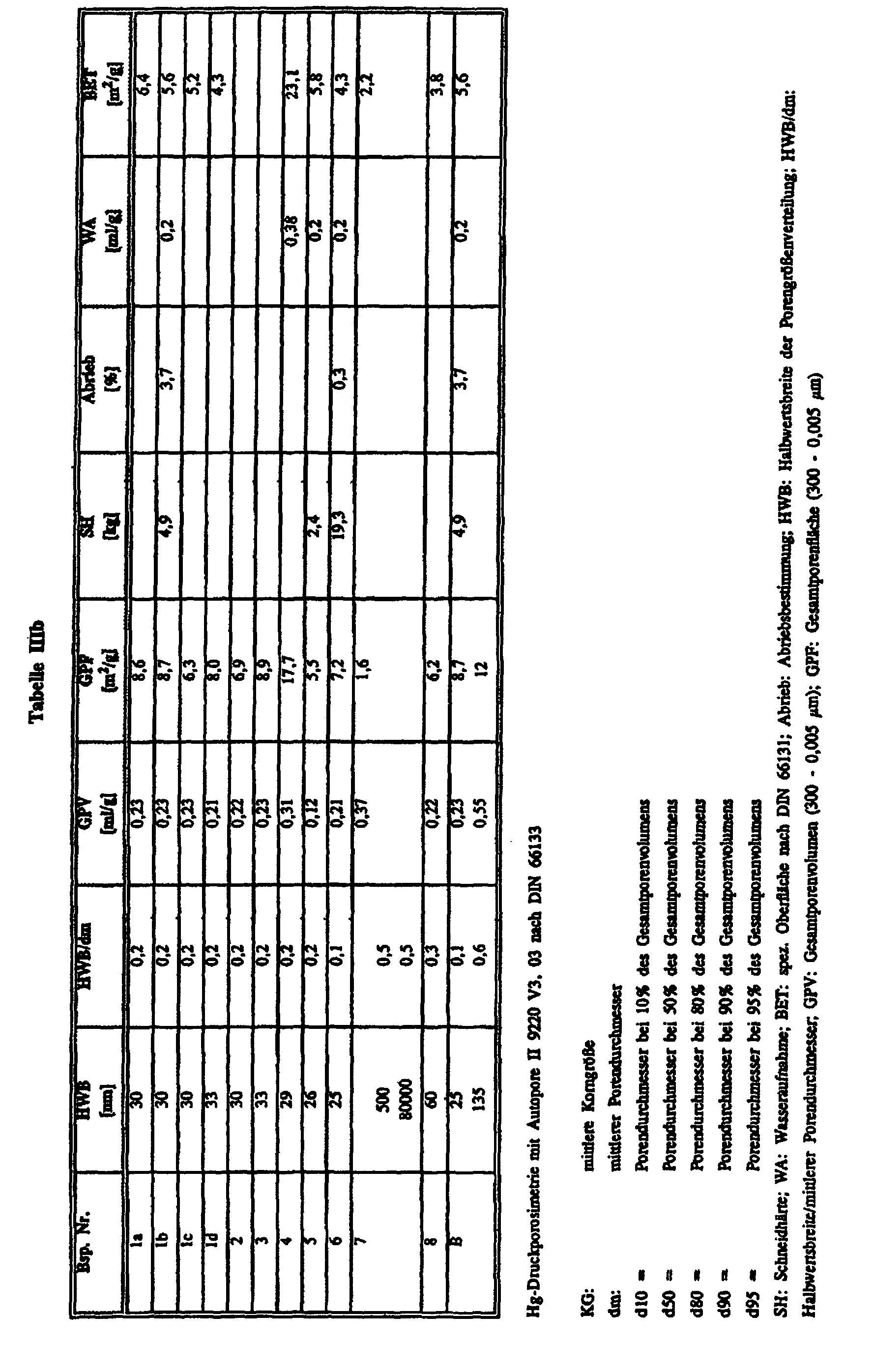

### Vergleichsbeispiel A

586 g Si₃N₄ (HC STARCK, LC 12) wurden mit 32 g Y₂O₃ (HC STARCK, grade fine) und 32 g α-Al₂O₃ (ALCOA, CT3000SG) wurden mit 150 g H₂O und 48 g HNO₃ 35 Minuten verknetet und bei 45 bis 150°C zu 4 mm Strängen extrudiert.

Die Stränge wurden bei 600, 800 und 1100°C an Luft 2h calciniert. Dabei zerfielen die Stränge zu Pulver (Schneidhärte = 0 kg).

### Vergleichsbeispiel B

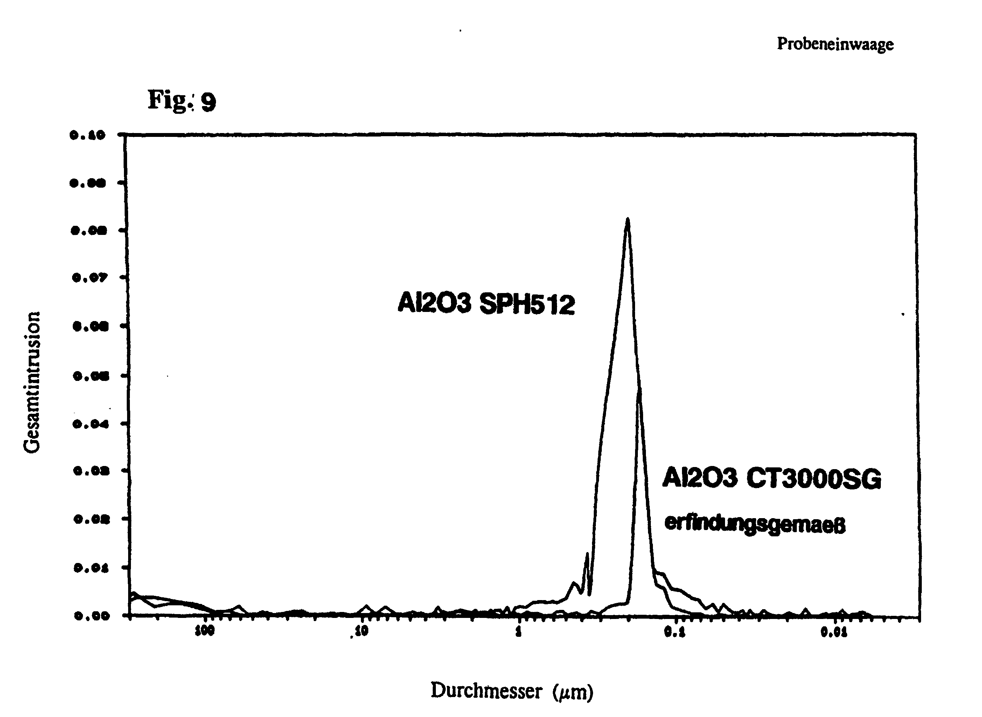

Der käufliche α-Al₂O₃-Katalysatorträger SPH512 der Firma Rhone Poulenc hat bei einer vergleichbaren BET von 5,4 m²/g und einem Gesamtporenvolumen (GPV) von 0,51 ml/g eine wesentlich breitere Porenverteilung (siehe Abb. 9) als der erfindungsgemäße Al₂O₃-Katalysatorträger CT3000SG von ALCOA in Tabelle IIIa und IIIb, Beispiel Nr. 1b und Abb. 1b.

### Vergleichsbeispiel C

ZrO₂-Träger hat nach herkömmlichem Verkneten und Verstrangen eine geringere Härte.

300 g ZrO₂-Pulver (Ausgangsprodukt aus Beispiel 5) der Firma TOSOH, TZ-3YS, und 9 g Verstrangungshilfsmittel wurden mit 31 ml Wasser versetzt, in einem Kneter für 2,5h verdichtet und anschließend im Extruder zu 3 mm Vollsträngen verformt. Die Stränge wurden 2h bei 120°C getrocknet und anschließend 2h bei 800°C unter Luft calciniert (gleiche Calciniertemperatur wie bei Beispiel 5).

An den Vollsträngen wurden folgende Eigenschaften gemessen:

| **Beispiel** | C | 5 |
|---|---|---|
| **Schneidhärte [kg]** | 0,6 | 2,4 |
| **BET-Oberfläche [m**^{**2**}**/g]** | 5,8 | 5,8 |
| **Wasseraufnahme [ml/g]** | 0,22 | 0,22 |
| **Stampfgewicht [g/ml]** | 1,455 | 1,543 |

### Vergleichsbeispiel D

### Geringere Härte eines herkömmlichen ZrO₂-Trägers auch bei hohen Calciniertemperaturen.

100 g oberflächenreiches Zr(OH)₄ (BET: 310 m²/g) wurde mit 3 g Verstrangungshilfsmittel und 45 ml Wasser versetzt und 1,5h im Kneter verdichtet. Verstrangungsversuche zur Formgebung dieses ZrO₂-Trägers gelangen nicht, da die Stränge schon beim Trocknen wieder zu Pulver zerfielen (Schneidhärte = 0 kg). Daher wurde die unverstrangte Knetmasse 2h bei 500°C calciniert. Die BET-Oberfläche des Pulvers nach der Calcinierung bei 500°C betrug 69,8 m²/g. Nach der Calcinierung (2h bei 800°C) ergab sich eine BET-Oberfläche von 11,2 m²/g.

### Beispiel Nr. 9 (instationärer Deacon-Prozeß)

### Katalysator 1

### Cu-K-Fe-Na auf Si₃N₄-Träger

### Herstellung des Si₃N₄-Trägers

900 g Si₃N₄ (HC STARCK; LC12) wurden mit 50 g Y₂O₃ (HC STARCK; grade fine) und 50 g Al₂O₃ (ALCOA; CT3000SG) mit einem Binder auf Polyacetalbasis, bestehend aus 276 g Polyoxymethylencopolymer (POM/PBDF) aus Trioxan und 2,5 Gew.-% Butandiolformal mit einem durchschnittlichen Molekulargewicht von 150.000, sowie mit 69 g Polybutandiolformal mit einem Molekulargewicht von 50.000 und 50 g Polyethylenglykol mit einem Molekulargewicht von 800 als Hilfsmittel, bei 180°C verknetet, granuliert und in einem Muffelofen unter N₂ bei 600°C 2h pyrolysiert und bei 1000°C 2h vorgesintert. Man erhielt einen Si₃N₄-Träger mit einer BET-Oberfläche von 22,3 m²/g und einer Wasseraufnahme von 0,608 ml/g.

### Tränkung des Si₃N₄-Trägers

111,5 g Si₃N₄-Granulat wurden zweimal jeweils mit 67,8 ml einer Lösung aus 7,06 g CuCl₂*2H₂O, 5,58 g KCI, 5,58 g NaCl und 9,29 g FeCl₃*6H₂O gelöst in destilliertem Wasser (Gesamtlösung 135,6 ml) imprägniert, 16h bei 120°C (nach jedem Tränkschritt) getrocknet und 3h bei 450°C calciniert. Man erhielt braunen Splitt mit einer BET-Oberfläche von 9,93 m²/g. Das Stampfgewicht betrug 0,978 g/ml (0,5 bis 1 mm Splitt).

### Nachtränkung mit einer FeCl₃-NaCl-Lösung

25 g des zuvor getränkten Si₃N₄-Trägers wurden mit einer Lösung aus 1,39 g NaCl und 2,21 g FeCl₃*6H₂O in 15,9 ml Wasser einmal imprägniert, 16h bei 120°C getrocknet und 3h bei 450°C calciniert. Das Stampfgewicht des Katalysators betrug 0,995 g/ml (0,5 bis 1 mm Splitt). Der Katalysator enthielt 1,8 Gew.-% Kupfer und 3 Gew.-% Eisen.

### Katalysator 2

### Cu-K-Fe-Na auf Si₃N₄-Träger

### Herstellung des Si₃N₄-Trägers

1000 g SiC (HC STARCK; UF15) wurden mit einem Binder auf Polyacetalbasis, bestehend aus 281 g Polyoxymethylencopolymer (POM/PBDF) aus Trioxan und 2,5 Gew.-% Butandiolformal mit einem durchschnittlichen Molekulargewicht von 150.000, sowie mit 70 g Polybutandiolformal mit einem Molekulargewicht von 50.000 und 50 g Polyethylenglykol mit einem Molekulargewicht von 800 als Hilfsmittel, bei 180°C verknetet, auf einem Walzenstuhl zu einem 0,5 mm dicken Fell aufgeschmolzen, zu Plätzchen zerkleinert und diese in einem Drehrohrofen bei 600°C 2h unter N₂ pyrolysiert und vorgesintert. Man erhielt einen SiC-Träger mit einer BET-Oberfläche von 22,3 m²/g und einer Wasseraufnahme von 0,35 ml/g.

### Tränkung des SiC-Trägers

150 g SiC-Plätzchen wurden zweimal jeweils mit 53 ml einer Lösung aus 23,7 g CuCl₂*2H₂O, 10,38 g KCI, 42, 78 g FeCl₃*6H₂O und 9,26 g NaCl gelöst in destilliertem Wasser (Gesamtlösung 106 ml) imprägniert, 16h bei 120°C (nach jedem Tränkschritt) getrocknet und 3h bei 450°C calciniert.

### Nachtränkung mit einer FeCl₃-NaCl-Lösung

70 g des zuvor getränkten SiC-Plätzchen wurden mit 22ml einer Lösung aus 3,92g NaCl und 18,22g FeCl₃*6H₂O gelöst in destilliertem Wasser (Gesamtlösung 25ml einmal imprägniert, 16h bei 120°C getrocknet und 3h bei 450°C calciniert. Man erhielt rotbraune Plätzchen der Zusammensetzung 3,3 Gew.-% Kupfer und 7,8 Gew.-% Eisen.

### Allgemeine Vorschrift zur instationären Chlorherstellung

In einem beheizten Röhrenreaktor mit 20 ml Katalysator-Festbett-Schüttung wurde eine Splittfraktion von 0,5 bis 1 mm eingefüllt. Nach der Beladephase mit einem trockenen HCl-Strom und anschließender Spülphase mit Inertgas (N₂ oder CO₂) wurde mit Luft oder reinem Sauerstoff regeneriert (dechloriert). Dieser Zyklus wurde wiederholt.

Durch eine on-line IR-Analytik wurde die HCl-Konzentration und durch eine on-line UV-Analytik wurde die Chlorkonzentration mit hoher Zeitauflösung kontinuierlich gemessen. Die integrale Chlormenge, die während der Dechlorierung freigesetzt wurde, konnte zur Kontrolle zusätzlich noch naßchemisch (jodometrisch) ermittelt werden.

Die Ergebnisse sind in den Tabellen 1 und 2 zusammengefaßt.

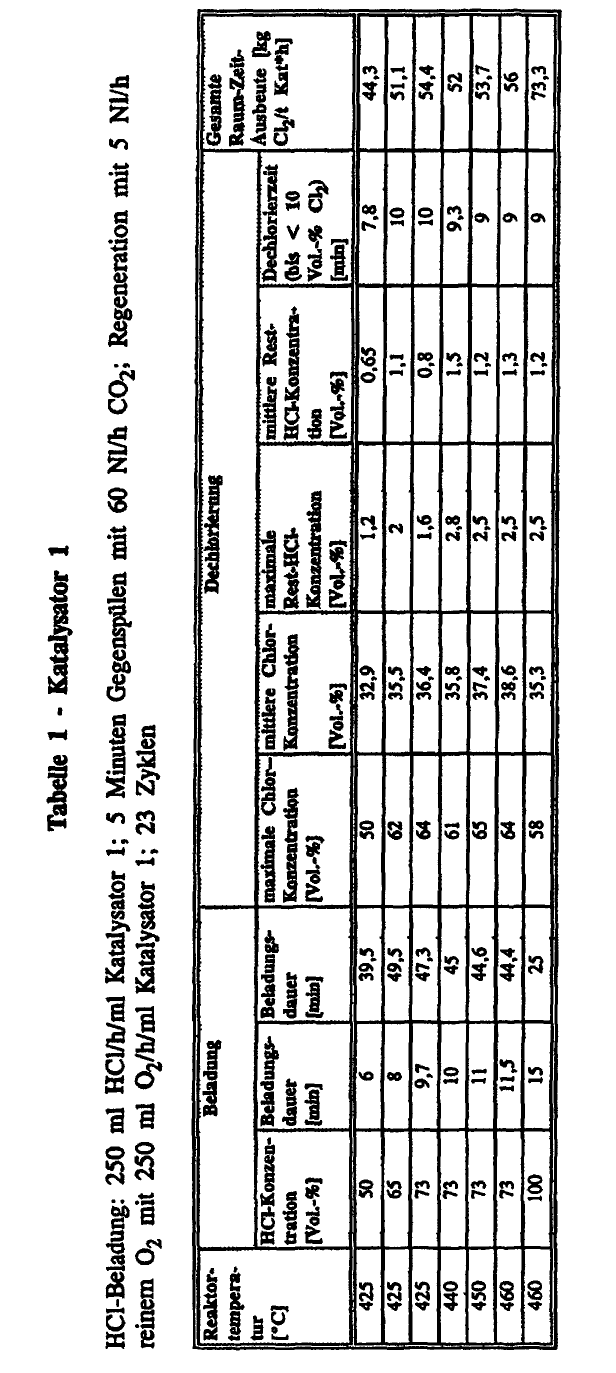

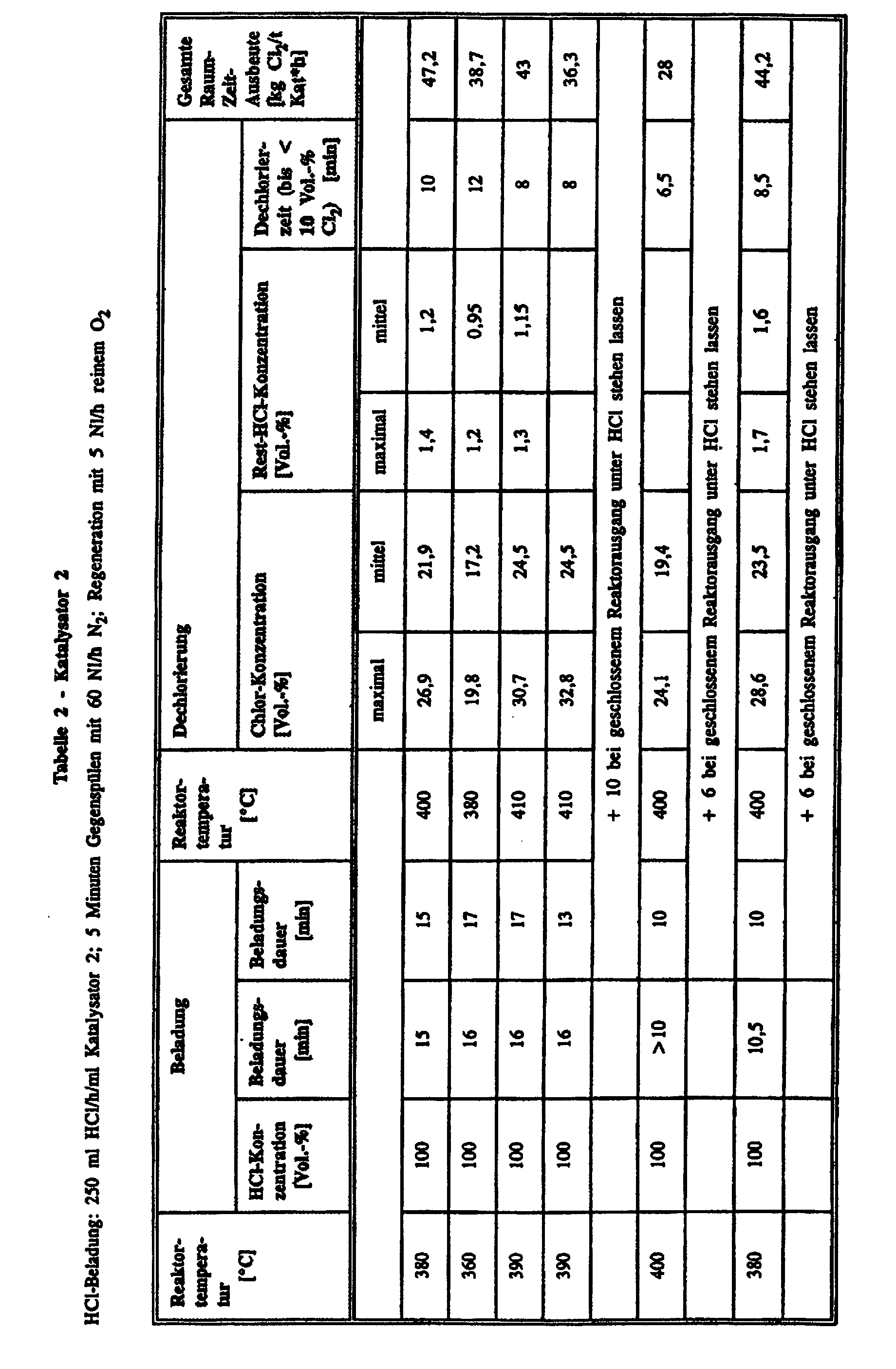

### Vergleichsbeispiel E [instationärer Deacon-Prozeß]

### Vergleichskatalysator

### (Cu-Fe-K auf Al₂O₃-Träger)

200 g Al₂O₃ (Pural SCF Ringe) wurden mit 92 ml einer Lösung aus 32,16 g CuCl₂*2H₂O, 58 g FeCl₃*6H₂O, 30 g KCI und 114 ml Wasser getränkt (Wasseraufnahme: 0,46 ml/g), 16h bei 120°C getrocknet, 3h bei 450°C calciniert und anschließend mit den restlichen 85 ml der Lösung getränkt, 16h bei 120°C getrocknet und 3h bei 450°C calciniert. Der Vergleichskatalysator C enthielt 3,8 Gew.-% Cu und 4,5 Gew.-% Fe; Stampfgewicht: 0,974 g/ml (9,5 bis 1 mm Splitt); BET-Oberfläche: 68,6 m²/g.

Beim Versuch, den Kontakt ein drittes Mal zu tränken, zerfiel dieser.

### Cu-Fe-K auf Al₂O₃-Träger

Analog der allgemeinen Vorschrift zur instationären Chlorherstellung der Beispiele 1 bis 3 wurde der Vergleichskatalysator Cu-Fe-K auf Al₂O₃ bei 365°C und HCl-Gasflüssen zwischen 4 bis 5 Nl/h mit 25% HCl (höhere HCl-Konzentrationen hielt der Träger nicht aus) bei einer HCl-Durchbruchszeit von 10 bis 14 Minuten beladen. Die Dechlorierung erfolgte mit 20 Nl/h Luft bei einer Regeneriertemperatur von 365°C mit Dechlorierungszeiten von 60 Minuten und einer integralen Chlormenge von 0,9 g, woraus sich eine Raum-Zeit-Ausbeute von 34 kg Chlor/t Kat.*h ergab.

Erfolgte die Dechlorierung mit 20 Nl/h Luft bei einer Regeneriertemperatur von 380°C, wurden Dechlorierungszeiten von 35 Minuten und eine integrale Chlormenge von 0,7 g gefunden, woraus sich eine Raum-Zeit-Ausbeute von 38 kg Chlor/t Kat.*h ergab.

Bei einer Reaktortemperatur von 400°C bei der Beladung und bei der Dechlorierung erhielt man eine maximale Chlorkonzentration von 8 Vol.-% Cl₂ und eine mittlere Chlorkonzentration von 4 Vol.-% Cl₂ bei Dechlorierzeiten (bis < 2 Vol.-% Chlor) von 25 Minuten. Die freigesetzte Chlormenge betrug integral 1 g. Die maximal gemessene Raum-Zeit-Ausbeute betrug 40 kg Chlor/t Kat.*h.

## Patentansprüche

1. Monomodale oder polymodale Katalysatorträger oder Katalysatoren, die eine spezifische Oberfläche nach BET von 0,01 bis 250m²/g, ein Porenvolumen von 0,05 bis 5 ml/g eine Schneidhärte von 1 bis 8 kg bei bei 800°C vorgesinterten Proben und eine monomodale oder eine polymodale Porengrößenverteilung mit einem mittleren Porendurchmesser von 50 bis 300.000 nm gemessen mit der Hg-Druckporosimetrie-Methode aufweisen und
a) 10 bis 95% des Porenvolumens bei dem 0,2- bis 100-fachen und/oder
b) 10 bis 80% des Porenvolumens bei dem 0,8- bis 100-fachen und/oder
c) 50 bis 95% des Porenvolumens bei dem 0,2- bis 1-fachen und/oder
d) 50 bis 80% des Porenvolumens bei dem 0,8- bis 1-fachen des mittleren Porendurchmessers liegt und
e) die Halbwertsbreite der Porengrößenverteilung weniger als das 0,6-fache des modalen Porendurchmessers beträgt.

2. Verfahren zur Herstellung von Katalysatorträgern oder Katalysatoren, nach Anspruch 1, durch Verformen eines Gemisches aus
A) 15 bis 70 Vol.-%
I) eines anorganischen Pulvers ausgewählt aus der Gruppe der Oxide, Nitride, Carbide, Silikate, Alumosilikate der Elemente Beryllium, Magnesium, Calcium, Strontium, Barium, Bor, Aluminium, Gallium, Indium, Thallium, Silicium, Germanium, Zinn, Blei, Antimon, Selen, Tellur, Polonium, Astat, Eisen, Cobalt, Nickel, Ruthenium, Rhodium, Palladium, Osmium, Iridium, Platin, Kupfer, Silber, Gold, Zink, Cadmium, Quecksilber, Scandium, Yttrium, Lanthan, Actinium, Titan, Zirkon, Hafnium, Vanadin, Niob, Tantal, Chrom, Molybdän, Wolfram, Mangan, Technetium, Rhenium, Cer, Praseodym oder deren Gemische und/oder
II) eines metallischen Pulvers ausgewählt aus Metallen und Legierungen der Elemente Bor, Aluminium, Gallium, Indium, Thallium, Silicium, Germanium, Zinn, Blei, Antimon, Selen, Tellur, Polonium, Neodym, Samarium, Dysprosium, Astat, Eisen, Cobalt, Raney-Cobalt, Nickel, Raney-Nickel, Ruthenium, Rhodium, Palladium, Osmium, Iridium, Platin, Kupfer, Silber, Gold, Zink, Cadmium, Quecksilber, Scandium, Yttrium, Lanthan, Actinium, Titan, Zirkon, Hafnium, Vanadin, Niob, Tantal, Chrom, Molybdän, Wolfram, Mangan, Technetium, Rhenium, Cer, Praseodym, WC, TiC, TaC, VC oder deren Gemische, WC-Cobalt, TiC-Cobalt, TaC-Cobalt, VC-Cobalt oder deren Gemische sowie Kohlenstoff und/oder
III) einer Alctivkomponente ausgewählt aus der Gruppe der anorganischen Säuren, der Metalle ausgewählt aus Lithium, Natrium, Kalium, Rubidium, Caesium, Francium, Beryllium, Magnesium, Calcium, Strontium, Barium, Bor, Aluminium, Gallium, Indium, Thallium, Silicium, Germanium, Zinn, Blei, Arsen, Antimon, Wismut, Selen, Tellur, Polonium, Astat, Eisen, Cobalt, Raney-Cobalt, Nikel, Raney-Nickel, Ruthenium, Rhodium, Palladium, Osmium, Iridium, Platin, Kupfer, Silber, Gold, Zink, Cadmium, Quecksilber, Scandium, Yttrium, Lanthan, Actinium, Titan, Zirkon, Hafnium, Vanadin, Niob, Tantal, Chrom, Molybdän, Wolfram, Mangan, Technetium, Rhenium, Cer, Praseodym, deren Gemische, oder deren Boraten, Carbonaten, Silikaten, Nitraten, Phosphaten, Arsenaten, Antimonaten, Bismutaten, Sulfaten, Selenaten, Telluraten, Vanadaten, Molybdaten, Niobaten, Chromaten, Oxiden, Hydroxiden, Halogeniden, Sulfiden, Seleniden, Telluriden, Nitriden, Phosphiden, Arseniden, Acetate, Acetylacetonate, Palladate, Platinate, Cyanide, Rhodanide, Manganate, Rhenate, Osmate, Carbiden, Siliciden, Boriden, deren Ammoniumverbindungen oder deren Gemische und/oder
IV) eines organischen Pulvers ausgewählt aus der Gruppe Teflon oder Polyimid
B) 30 bis 85 Vol.-% eines Bindemittels, ausgewählt aus einem Polyethylen- oder Polyporpylenpolymer oder einem Copolymeren aus Ethylen, Propylen, buten-1 oder Isobuten oder einem Polystyrolcopolymer oder einem Polymethylmethacrylatcopolymer oder einem Polyethylenoxidcopolymer oder einem Ethylenvinylacetatcopolymer oder einer Mischung aus
B₁) 50 bis 100 Gew.-% eines Polyoxymethylenhomo- oder copolymerisats und
B₂) 0 bis 50 Gew.-% eines in B₁) homogen gelösten oder mit einer mittleren Teilchengröße von weniger als 1µm in B₁) dispergierten Polymerisats und
C) 0 bis 15 Vol.-% eines Dispergierhilfsmittels,
entfernen des Bindemittels und anschließendes Vorsintern und gegebenenfalls Aufbringen von Aktivkomponenten III auf der Komponente A) oder auf der vorgesinterten Masse durch gegebenenfalls mehrfaches Tränken, Imprägnieren, Sprühimprägnieren, Auffällen, Hicoaten, Washcoaten oder Sprühtrocknen, **dadurch gekennzeichnet, daß** die Komponente A) ein nanokristallines Pulver mit einer mittleren Korngröße von 5 nm bis 500.000 nm ist, wobei die Abweichungen der Korngröße bei 80% der Körner 0 bis 30% der mittleren Korngröße betragen und das Bindemittel durch Pyrolyse bei Temperaturen von 300 bis 600°C entfernt wird und anschließend bei Temperaturen von 600 bis 1400°C vorgesintert wird und die Katalysatorträger oder Katalysatoren nach der pyrolytischen Entfernung des Binders eine spezifische Oberfläche nach BET von 0,01 bis 250 m²/g und eine Porengrößenverteilung von 50 bis 300.000 nm gemessen mit der Hg-Druckporosimetrie-Methode aufweisen.

3. Verfahren nach Anspruch 2, **dadurch gekennzeichnet, daß** man Pulver mit polymodaler Korngrößenverteilung oder mit innerer Porosität einsetzt.

4. Verfahren nach Anspruch 2 oder 3, **dadurch gekennzeichnet, daß** man als Metall Aluminium, Eisen, Cobalt, Nickel, Palladium, Platin, Kupfer, Silber, Molybdän, Zink, Titan, Zirkon, Wolfram, Niob, Chrom oder Kohlenstoff oder als anorganisches Pulver Al₂O₃, MgO, SiO₂, TiO₂, Y₂O₃, ZrO₂, ZnO, Fe₃O₄, Fe₂O₃, CoO, Co₂O₃, Cr₂O₃, NiO, B₂O₃, Ce₂O₃, CeO₂, Pr₂O₃, B₄C, SiC, WC, TiC, TaC, Si₃N₄, AlN, BN, TiN, ZrN, oder deren Gemische einsetzt.

5. Verfahren nach einem der Ansprüche 2 bis 4, **dadurch gekennzeichnet, daß** man als Metall Eisen, Cobalt, Nickel, Chrom, Molybdän, Titan oder als anorganisches Pulver SiC, Si₃N₄, BN, B₄C, WC, TiC, TiN, ZrN und AlN, oder deren Gemische einsetzt.

6. Verfahren nach einem der Ansprüche 2 bis 5, **dadurch gekennzeichnet, daß** man als anorganisches Pulver SiC, Si₃N₄ oder deren Gemische einsetzt.

7. Verfahren nach einem der Ansprüche 2 bis 6, **dadurch gekennzeichnet, daß** man die Mischung aus A), B) und C) durch Granulieren, Pressen, Walzen, Extrusion, Spritzguß oder Strangguß bei 80 bis 250°C verformt.

8. Verfahren nach einem der Ansprüche 2 bis 7, **dadurch gekennzeichnet, daß** man die Verformung so durchführt, daß diese als Schüttung von Einzelteilen oder als Monolithe in Form von Raschig-Ringen, Sattelkörpern, Sternringen, gelochten und/oder gerippten geometrischen Körpern wie Ringen, Kugeln, Quadern, Würfeln, Kegeln, Pyramiden, Prismen, Oktaedern, Zylindern, Pyramidenstümpfen und Kegelstümpfen, Wagenräderprofilen, Fensterrahmenprofilen oder Honigwabenprofilen, anfallen.

9. Verwendung eines Katalysatorträgers oder Katalysators gemäß Anspruch loder eines monomodalen oder polymodalen Katalysatorträgers oder Katalystors, hergestellt nach einem Verfahren gemäß einem der Ansprüche 2 bis 9, zur Chlorherstellung aus Chlorwasserstoff in einem instationären Deacon-Prozeß, zur Umsetzung von Ethylbenzol zu Styrol in einer instationären Oxy-Dehydrierung, zur Aziridinherstellung aus Ethanolamin, zur Umsetzung von Trimethylcyclohexenon zu Trimethylphenol bei Reduktionen, Hydrierungen, Oxidationen, Dehydrierungen, sauer oder basisch katalysierten Reaktionen oder Reaktionen in der Wirbelschicht, zur Entfernung von Verbrennungsrückständen aus Dieselabgasen und zur Entfernung von NOₓ aus Abgasen, in Bioreaktoren gemeinsam mit Bakterien und als Biokatalysatorträger mit immobilisierten Enzymen oder Mikroben.

## Claims

1. A monomodal or polymodal catalyst support or catalyst having a BET specific surface area of from 0.01 to 250 m²/g, a pore volume of from 0.05 to 5 ml/g, a cutting hardness. of from 1 to 8 kg for samples presintered at 800°C and a monomodal or polymodal pore size distribution having a mean pore diameter of from 50 to 300,000 nm measured by the mercury pressure porosimetry method, wherein
a) from 10 to 95 % of the pore volume is at from 0.2 to 100 times the mean pore diameter and/or
b) from 10 to 80 % of the pore volume is at from 0.8 to 100 times the mean pore diameter and/or
c) from 50 to 95 % of the pore volume is at from 0.2 to 1 times the mean pore diameter and/or
d) from 50 to 80 % of the pore volume is at from 0.8 to 1 times the mean pore diameter and
e) the width at half height of the pore size distribution is less than 0.6 times the modal pore diameter.

2. A process for producing a catalyst support or catalyst as claimed in claim 1 by shaping a mixture of
A) from 15 to 70 % by volume of
I) an inorganic powder selected from the group of oxides, nitrides, carbides, silicates, aluminosilicates of the elements beryllium, magnesium, calcium, strontium, barium, boron, aluminum, gallium, indium, thallium, silicon, germanium, tin, lead, antimony, selenium, tellurium, polonium, astatine, iron, cobalt, nickel, ruthenium, rhodium, palladium, osmium, iridium, platinum, copper, silver, gold, zinc, cadmium, mercury, scandium, yttrium, lanthanum, actinium, titanium, zirconium, hafnium, vanadinium, niobium, tantalum, chromium, molybdenum, tungsten, manganese, technetium, rhenium, cerium, praseodymium or mixtures thereof and/or
II) a metallic powder selected from among metals and alloys of the elements boron, aluminum, gallium, indium, thallium, silicon, germanium, tin, lead, antimony, selenium, tellurium, polonium, neodymium, samarium, dysprosium, astatine, iron, cobalt, Raney cobalt, nickel, Raney nickel, ruthenium, rhodium, palladium, osmium, iridium, platinum, copper, silver, gold, zinc, cadmium, mercury, scandium, yttrium, lanthanum, actinium, titanium, zirconium, hafnium, vanadinium, niobium, tantalum, chromium, molybdenum, tungsten, manganese, technetium, rhenium, cerium, praseodymium, WC, TiC, TaC, VC or mixtures thereof, WC-cobalt, TiC-cobalt, TaC-cobalt, VC-cobalt or mixtures thereof and also carbon and/or
III) an active component selected from the group of the inorganic acids, the metals selected from among lithium, sodium, potassium, rubidium, cesium, francium, beryllium, magnesium, calcium, strontium, barium, boron, aluminum, gallium, indium, thallium, silicon, germanium, tin, lead, arsenic, antimony, bismuth, selenium, tellurium, polonium, astatine, iron, cobalt, Raney cobalt, nickel, Raney nickel, ruthenium, rhodium, palladium, osmium, iridium, platinum, copper, silver, gold, zinc, cadmium, mercury, scandium, yttrium, lanthanum, actinium, titanium, zirconium, hafnium, vanadinium, niobium, tantalum, chromium, molybdenum, tungsten, manganese, technetium, rhenium, cerium, praseodymium, mixtures thereof, or their borates, carbonates, silicates, nitrates, phosphates, arsenates, antimonates, bismutates, sulfates, selenates, tellurates, vanadates, molybdates, niobates, chromates, oxides, hydroxides, halides, sulfides, selenides, tellurides, nitrides, phosphides, arsenides, acetates, acetylacetonates, palladates, platinates, cyanides, thiocyanates, manganates, rhenates, osmates, carbides, silicides, borides, their ammonium compounds or their mixtures and/or
IV) an organic powder selected from the group teflon or polyimide
B) from 30 to 85 % by volume of a binder selected from among polyethylene and polypropylene polymers and copolymers of ethylene, propylene, 1-butene or isobutene and polystyrene copolymers and polymethyl methacrylate copolymers and polyethylene oxide copolymers and ethylene-vinyl acetate copolymers and mixtures of
B₁) from 50 to 100 % by weight of a polyoxymethylene homopolymer or copolymer and
B₂) from 0 to 50 % by weight of a polymer homogeneously dissolved in B₁) or dispersed in B₁) at a mean particle size of less than 1 µm and
C) from 0 to 15 % by volume of a dispersant,
removing the binder and subsequent presintering and, if desired, applying active components III to the component A) or to the presintered composition by single or multiple steeping, impregnation, spray impregnation, precipitating on, hicoating, washcoating or spray drying, wherein the component A) is a nanocrystalline powder having a mean particle size of from 5 nm to 500, 000 nm, with the deviation of the particle size at 80% of the particles being from 0 to 30% of the mean particle size, and the binder is removed by pyrolysis at from 300 to 600°C and the shaped body or bodies is/are subsequently presintered at from 600 to 1400°C and the catalyst support or catalyst after the pyrolytic removal of the binder has a BET specific surface area of from 0.01 to 250 m²/g and a pore size distribution of from 50 to 300,000 nm measured by the mercury pressure porosimetry method.

3. A process as claimed in claim 2, wherein powder having a polymodal particle size distribution or having internal porosity is used.

4. A process as claimed in claim 2 or 3, wherein aluminum, iron, cobalt, nickel, palladium, platinum, copper, silver, molybdenum, zinc, titanium, zirconium, tungsten, niobium, chromium or carbon are used as metal or Al₂O₃, MgO, SiO₂, TiO₂, Y₂O₃, ZrO₂, ZriO, Fe₃O₄, Fe₂O₃, CoO, Co₂O₃, Cr₂O₃, NiO, B₂O₃, Ce₂O₃, CeO₂, Pr₂O₃, B₄C, SiC, WC, TiC, TaC, Si₃N₄, AlN, BN, TiN, ZrN or. a mixture thereof is used as inorganic powder.

5. A process as claimed in any of claims 2 to 4, wherein iron, cobalt, nickel, chromium, molybdenum, titanium is used as metal or SiC, Si₃N₄, BN, B₄C, WC, TiC, TiN, ZrN or AlN or a mixture thereof is used as inorganic powder.

6. A process as claimed in any of claims 2 to 5, wherein SiC, Si₃N₄ or a mixture thereof is used as inorganic powder.

7. A process as claimed in any of claims 2 to 6, wherein the mixture of A), B) and C) is shaped by granulation, pressing, rolling, extrusion, injection molding or continuous casting at from 80 to 250°C.

8. A process as claimed in any of claims 2 to 7, wherein the shaping is carried but in such a way that the catalyst support or catalyst is obtained as a bed of individual parts or as monolith in the form of Raschig rings, saddles, star rings, perforated and/or ribbed geometric bodies such as rings, spheres, cuboids, cubes, cones, pyramids, prisms, octahedra, cylinders, truncated pyramids or truncated cones, wagon wheel profiles, window frame profiles or honeycomb profiles.

9. Use of a catalyst support or catalyst as claimed in claim 1 or of a monomodal or polymodal catalyst support or catalyst produced by a process as claimed in any of claim 2 to 9 for preparing chlorine from hydrogen chloride in a non-steady-state Deacon process, for the reaction of ethylbenzene to give styrene in a non-steady-state oxydehydrogenation, for preparing aziridine from ethanolamine, for the reaction of trimethylcyclohexenone to give trimethylphenol, in reductions, hydrogenations, oxidations, dehydrogenations, acid- or base-catalyzed reactions or reactions in a fluidized bed, for removing combustion residues from diesel exhaust gases and for removing NOₓ from waste gases, in bioreactors together with bacteria and as biocatalyst supports with immobilized enzymes or microbes.

## Revendications

1. Supports de catalyseurs ou catalyseurs monomodes ou polymodes, présentant une surface spécifique selon BET de 0,01 à 250 m²/g, un volume porique de 0,05 à 5 ml/g, une dureté de coupe de 1 à 8 kg pour des échantillons préfrittés à 800°C et une distribution porométrique monomodale ou polymodale avec un diamètre de pores moyen de 50 à 300.000 nm, mesuré par la méthode de porosimétrie à pression de Hg, et
a) de 10 à 95% du volume porique se situent dans la plage de 0,2 à 100 fois et/ou
b) de 10 à 80% du volume porique se situent dans la plage de 0,8 à 100 fois et/ou
c) de 50 à 95% du volume porique se situent dans la plage de 0,2 à 1 fois et/ou
d) de 50 à 80% du volume porique se situent dans la plage de 0,8 à 1 fois le diamètre moyen des pores et
e) la largeur de valeur moyenne de la distribution porométrique est inférieure à 0,6 fois le diamètre de pore modal.

2. Procédé de préparation de supports de catalyseurs ou de catalyseurs, selon la revendication 1, par façonnage d'un mélange constitué de
A) 15 à 70% en volume
I) d'une poudre inorganique choisie dans le groupe des oxydes, nitrures, carbures, silicates, aluminosilicates des éléments béryllium, magnésium, calcium, strontium, baryum, bore, aluminium, gallium, indium, thallium, silicium, germanium, étain, plomb, antimoine, sélénium, tellure, polonium, astate, fer, cobalt, nickel, ruthénium, rhodium, palladium, osmium, iridium, platine, cuivre, argent, or, zinc, cadmium, mercure, scandium, yttrium, lanthane, actinium, titane, zirconium, hafnium, vanadium, niobium, tantale, chrome, molybdène, tungstène, manganèse, technétium, rhénium, cérium, praséodyme ou leurs mélanges, et / ou
II) une poudre métallique choisie parmi des métaux et alliages des éléments bore, aluminium, gallium, indium, thallium, silicium, germanium, étain, plomb, antimoine, sélénium, tellure, polonium, néodyme, samarium, dysprosium, astate, fer, cobalt, cobalt de Raney, nickel, nickel de Raney, ruthénium, rhodium, palladium, osmium, iridium, platine, cuivre, argent, or, zinc, cadmium, mercure, scandium, yttrium, lanthane, actinium, titane, zirconium, hafnium, vanadium, niobium, tantale, chrome, molybdène, tungstène, manganèse, technétium, rhénium, cérium, praséodyme, WC, TiC, TaC, VC ou leurs mélanges, WC - cobalt, TiC - cobalt, TaC - cobalt, VC - cobalt ou leurs mélanges, ainsi que le carbone, et / ou
III) un composant actif choisi dans le groupe des acides inorganiques, des métaux choisis parmi le lithium, le sodium, le potassium, le rubidium, le césium, le francium, le béryllium, le magnésium, le calcium, le strontium, le baryum, le bore, l'aluminium, le gallium, l'indium, le thallium, le silicium, le germanium, l'étain, le plomb, l'arsenic, l'antimoine, le bismuth, le sélénium, le tellure, le polonium, l'astate, le fer, le cobalt, le cobalt de Raney, le nickel, le nickel de Raney, le ruthénium, le rhodium, le palladium, l'osmium, l'iridium, le platine, le cuivre, l'argent, l'or, le zinc, le cadmium, le mercure, le scandium, l'yttrium, le lanthane, l'actinium, le titane, le zirconium, l'hafnium, le vanadium, le niobium, le tantale, le chrome, le molybdène, le tungstène, le manganèse, le technétium, le rhénium, le cérium, le praséodyme, leurs mélanges, ou leurs borates, carbonates, silicates, nitrates, phosphates, arséniates, antimoniates, bismuthates, sulfates, séléniates, tellurates, vanadates, molybdates, niobates, chromates, oxydes, hydroxydes, halogénures, sulfures, séléniures, tellurures, nitrures, phosphures, arséniures, acétates, acétyl-acétonates, palladates, platinates, cyanures, sulfocyanates, manganates, rhénates, osmiates, carbures, siliciures, borures, leurs composés d'ammonium ou leurs mélanges, et/ou,
IV) une poudre organique choisie dans le groupe du Téflon ou du polyimide,
B) 30 à 85% en volume d'un liant, choisi parmi un polymère de polyéthylène ou de polypropylène ou un copolymère d'éthylène, propylène, butène-1 ou isobutène, ou un copolymère de polystyrène ou un copolymère de poly(méthacrylate de méthyle) ou un copolymère de poly(oxyde d'éthylène) ou un copolymère de poly(vinylacétate d'éthylène) ou un mélange constitué de
B₁) 50 à 100% en poids d'un homopolymère ou d'un copolymère de polyoxyméthylène et
B₂) 0 à 50% en poids d'un polymère dissous de manière homogène dans B₁) ou dispersé dans B₁) avec une taille moyenne de particules de moins de 1 µm et
C) 0 à 15% en volume d'un adjuvant de dispersion,
élimination du liant et préfrittage subséquent, et éventuellement application de composant actif III sur le composant A) ou sur la masse préfrittée par des opérations éventuellement multiples d'imbibition, d'imprégnation, d'imprégnation par pulvérisation, de précipitation, d'imprégnation Hicoat ou washcoat ou de séchage à pulvérisation,
**caractérisé en ce que** le composant A) est une poudre nanocristalline d'une granulométrie moyenne de 5 nm à 500.000 nm, les écarts de granulométrie étant, pour 80% des grains, de 0 à 30% de la granulométrie moyenne, et **en ce que** le liant est éliminé par pyrolyse à des températures de 300 à 600°C et que l'on procède ensuite à un préfrittage à des températures de 600 à 1400°C et que les supports de catalyseurs ou les catalyseurs présentent, après l'élimination pyrolytique du solvant, une surface spécifique selon BET de 0,01 à 250 m²/g et une distribution de la taille des pores de 50 à 300.000 nm, mesurée par la méthode de porosimétrie à mercure.

3. Procédé selon la revendication 2, **caractérisé en ce que** l'on met en oeuvre une poudre à distribution granulométrique polymodale ou à porosité interne.

4. Procédé selon la revendication 2 ou 3, **caractérisé en ce que** l'on met en oeuvre en tant que métal de l'aluminium, du fer, du cobalt, du nickel, du palladium, du platine, du cuivre, de l'argent, du molybdène, du zinc, du titane, du zirconium, du tungstène, du niobium, du chrome ou du carbone, ou en tant que poudre inorganique Al₂O₃, MgO, SiO₂, TiO₂, Y₂O₃, ZrO₂, ZnO, Fe₃O₄, Fe₂O₃, CoO, Co₂O₃, Cr₂O₃, NiO, B₂O₃, Ce₂O₃, Pr₂O₃, B₄C, SiC, WC, TiC, TaC, Si₃N₄, AIN, BN, TiN, ZrN, ou leurs mélanges.

5. Procédé selon l'une des revendications 2 à 4, **caractérisé en ce que** l'on met en oeuvre en tant que métal du fer, du cobalt, du nickel, du chrome, du molybdène, du titane, ou en tant que poudre inorganique SiC, Si₃N₄, BN, B₄C, WC, TiC,TiN, ZrN et AIN, ou leurs mélanges.

6. Procédé selon l'une des revendications 2 à 5, **caractérisé en ce que** l'on met en oeuvre en tant que poudre inorganique SiC, Si₃N₄ ou leurs mélanges.

7. Procédé selon l'une des revendications 2 à 6, **caractérisé en ce que** le mélange de A), B) et C) est façonné par granulation, pressage, laminage, extrusion, moulage par injection ou boudinage à une température de 80 à 250°C.

8. Procédé selon l'une des revendications 2 à 7, **caractérisé en ce que** l'on entreprend le façonnage de manière à obtenir un vrac d'éléments individuels ou des monolithes en forme d'anneaux Raschig, de selles, d'anneaux en étoile, de corps géométriques perforés ou nervurés comme des anneaux, des billes, des parallélépipèdes, des cubes, des cônes, des pyramides, des prismes, des octaèdres, des cylindres, des pyramides tronquées et des cônes tronqués, des profilés en roues, en cadres de fenêtres ou en nid d'abeille.

9. Utilisation d'un support de catalyseur ou d'un catalyseur selon la revendication 1 ou d'un support de catalyseur ou d'un catalyseur monomodal ou polymodal préparé par un procédé selon l'une des revendications 2 à 9, pour la production de chlore à partir de chlorure d'hydrogène dans un procédé Deacon instationnaire, pour la conversion d'éthylbenzène en styrène par une déshydrogénation instationnaire, pour la préparation d'aziridine à partir d'éthanolamine, pour la conversion de triméthylcyclohexénone en triméthylphénol par des réductions, des hydrogénations, des oxydations, des déshydrogénations, des réactions à catalyse acide ou basique ou des réactions en lit fluidisé, pour l'élimination de résidus de combustion de gaz d'échappement de moteurs diesel et pour l'élimination de NOₓ de gaz d'échappement, dans des bioréacteurs conjointement avec des bactéries et comme support de biocatalyseur avec enzymes ou microbes immobilisés.
